(19)

**Europäisches Patentamt**

**European Patent Office**

**Office européen des brevets**

(11)  **EP 4 316 843 A1**

(12)  **EUROPEAN PATENT APPLICATION**

(43) Date of publication:
**07.02.2024 Bulletin 2024/06**

(21) Application number: **22306181.3**

(22) Date of filing: **03.08.2022**

(51) International Patent Classification (IPC):
*B33Y 70/00* (2020.01)   *B29C 64/106* (2017.01)
*C12M 1/26* (2006.01)   *C12M 1/00* (2006.01)
*C12M 3/00* (2006.01)

(52) Cooperative Patent Classification (CPC):
**C12M 33/00; B29C 64/371; B33Y 70/00;**
**C12M 23/26; C12M 23/48**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
- **SARTORIUS STEDIM FMT SAS**
  **13400 Aubagne (FR)**
- **UNIVERSITE CLAUDE BERNARD - LYON 1**
  **69100 Villeurbanne (FR)**
- **Institut National des Sciences Appliquees**
  **de Lyon (Insa Lyon)**
  **69100 Villeurbanne (FR)**
- **Centre National de la Recherche Scientifique**
  **(CNRS)**
  **75016 Paris (FR)**

- **Ecole Superieure de Chimie, Physique,**
  **Electronique**
  **de Lyon**
  **69616 Villeurbanne Cedex (FR)**

(72) Inventors:
- **Gay, Isabelle**
  **13124, Peypin (FR)**
- **Barbaroux, Magali**
  **13112, La Destrousse (FR)**
- **Dufour, Alexandre**
  **69006, Lyon (FR)**
- **Marquette, Christophe**
  **69100, Villeurbanne (FR)**
- **Petiot, Emma**
  **69100, Villeurbanne (FR)**

(74) Representative: **Derambure Conseil**
  **c/o Plasseraud IP**
  **66, rue de la Chaussée d'Antin**
  **75440 Paris Cedex 09 (FR)**

(54)  **METHOD OF ASSEMBLING A BIOREACTOR HAVING A BIOLOGICAL MATERIAL DEPOSITING END THAT IS MOVABLE WITH THE TOP PORTION TO ALLOW BIOPRINTING**

(57)   A bioreactor (4) allowing bioprinting inside an interior volume thereof is assembled to form a side wall (SW) extending upwardly from a base provided with a target support (20) for the bioprinting. Once an adapter (5) has been coupled to a bioreactor top portion (4b) to plug a corresponding aperture O4, a biological material depositing end (8) may be maintained in an inserted state, extending through and/or being adapted to be fed via the aperture (O4). A sealed enclosure (E), including the side wall and ports, is formed by a sealing operation to interconnect the base and the top portion (4b). Bioprinting is done by driving from outside the depositing end (8), using variation of said volume, for delivering the biological material on the support (20) and then, a culture and/or maturation phase of the printed tissue may start inside the enclosure (E), without displacement of the printed tissue.

FIG. 2

EP 4 316 843 A1

**Description**

FIELD OF THE INVENTION

The instant disclosure relates to systems and enclosures used for bioprinting. More particularly, the invention relates to a method of assembling a bioreactor and a bioreactor, typically a single-use bioreactor, suitable for bioprinting, while being also used for a subsequent step without moving the printout.

TECHNICAL BACKGROUND

The forming of three dimensional (3D) objects using biological material or the coating of such three-dimensional objects under sterile conditions, requires high effort to uphold the sterile conditions and to assemble the required apparatuses for forming or coating under sterile conditions. In the following, bioprinting is defined as the use of a 3D printing technology with material(s) that incorporate viable living cells, e.g. to produce tissue for reconstructive surgery.

A known 3D bioprinter includes a driving system with a frame, allowing movement along XYZ directions, a reservoir and a nozzle/needle having a dispenser form, with a biological material depositing end for discharging a biomaterial. To maintain sterile conditions, the bioprinter must be contained in a large printing chamber. Document US2022/0105686 A1 discloses a method of controlling a 3D bioprinter, in which a printing chamber is used for accommodating the bioprinter. Successive operations of printing living tissue in a sterile environment and ensuring $CO_2$ incubation may be performed in the printing chamber without moving a printout after the living tissue is printed. Then, the material is transferred for maturation, typically after introduction in a bioreactor, keeping sterile conditions.

Document WO 2022064125 A1 discloses a bioprinting process, in which an aseptic isolator is provided to delimit the bioprinting space. The printing part and an associated robot are housed in such bioprinting space. The aseptic isolator has a window allowing passage of a laser beam for scanning and activating the bioprinting source. A thermoregulated space for maturing tissue sheets and a sterile airlock for exchanges between the inside and the outside of said isolator are also included in the aseptic isolator.

Document EP 3194150 B1 discloses a sterilizable container, delimiting an interior volume with the printer assembly accommodated in such interior volume. The container may function as a bioreactor. To uphold the sterile conditions, the printing assembly can be removed by a transfer hatch formed on a side wall. The assembling of the printing assembly and the subsequent removing of such assembly imply complications. Document US 2020/0406542 discloses a bioprinting device, which can be interfaced laterally with a bioreactor, making a transfer possible without opening the door of the device housing delimiting the interior volume. A displacement is however required for the printout.

Current constructions of a sterilizable container forming a printing chamber are complex. More generally, while additive manufacturing of biological systems has the potential to revolutionize the engineering of soft structures, bioprosthetics, and scaffolds for tissue repair, there is a need to overcome some limitations. It would be of interest to keep cells alive during the whole process (bioprinting and then maturation), for instance using aqueous environments that are pH, ionic, temperature, and sterility controlled within tight tolerances. Expensive bioprinters that attempt to address such challenge have been tested but imply fastidious steps and effort to uphold the sterile conditions after a bioprinting step. There is therefore still a place for improvement, in the specific field of the disclosure.

OBJECTS AND SUMMARY

For improving situation, embodiments of the invention provide a method of assembling a bioreactor, which may be a single-use sterilizable bioreactor, allowing bioprinting inside an interior volume of the bioreactor using a biological material depositing end for delivering a biological material on a target support, the method comprising:

- providing the target support for the bioprinting, on or above a base portion (the base portion being adapted/intended to be the lower part of the bioreactor);
- coupling an adapter to the bioreactor at an aperture, for instance to a bioreactor top portion (which may be distinct from the base portion, while participating in delimiting the interior volume, directly by a top portion inner face), the aperture being hermetically plugged by the adapter;
- forming a side wall to delimit the interior volume of the bioreactor, with the side wall made integral with the base portion, the side wall being adapted to taper and/or extend longitudinally, upwardly from/away from the base portion in an upstanding configuration of the bioreactor;
- providing one or two connection ports for direct fluidic communication with the interior volume, in order to form a fluid inlet port and a fluid outlet port, so that the bioreactor is usable for a culture and/or maturation phase of the printed material (obtained by bioprinting), inside the interior volume, after the bioprinting; and
- forming a sealed enclosure including the side wall, by at least one sealing operation to interconnect the base portion and a/the top portion, with the sealed enclosure being at least partly flexible and movable in order to allow the biological material depositing end, inserted through or fed/filled (with biological material) via the aperture when secured to the adapter, to be movable along at least two transverse directions inside the interior volume.

No displacement is required for the printed material. Same container, here the bioreactor, can be used for all process steps from bioprinting to recovery of the final tissue, including cell culture (and possibly storage and shipment of the printed tissue). This facilitates upholding the sterile conditions, while the size and design of the bioreactor may easily vary. The sealed enclosure is compatible with bioprinting constraints.

Optionally, at least one functional component may be affixed to a wall member delimiting the interior volume, in a step performed prior to the assembling of all the wall members of the sealed enclosure. Such functional component(s) may include a stirring member, a PH and/or oxygen sensor, a temperature sensor, an aerator.

Besides in some options, before the bioprinting and after having the depositing end in fluid communication with a reservoir part (for instance a cartridge/syringe or the like) of the printer unit, an amount of product (biological material or similar) can be delivered inside the sealed enclosure using a collector, without reaching the target support, until a suitable flow rate is obtained for the flowable product to be used in the bioprinting phase. This corresponds to a calibration step, for instance using a piston, a screw system and/or a pneumatic tool of the printer unit.

The method provides a base portion that may be stationary, while the bioreactor extends upwardly. Thanks to such method, a first printing may occur inside the interior volume, targeting a given location, a culture cell is performed at same location based on the tissue obtained from the first printing, and then a second printing may possibly be started without any change of location. The base portion may optionally have a perimeter portion, of annular shape, that is rigid or rigidly secured to remain undeformed and without variation in the perimeter.

The adapter can be further movable along a third direction, typically perpendicular to the base portion (Z direction when the transverse directions are directions XY). The adapter may be arranged in the top portion, away from the side wall, for instance using a rigid spacing structure. In some options, the base portion may have a movable part displaced to get the target support closer to the adapter, possibly without longitudinally moving the top portion. Such latter option may be performed without any displacement of printed tissue relative to the target support, thus not altering fixing of the cells to the support arranged in the base portion.

An annular space may be available around the adapter, at least when the adapter is in a lowered position, thanks to spacing means included in or arranged around the top portion. The injection needle or similar depositing end may be movable along three orthogonal directions due to flexibility of the top portion, enabling bioprinting by the needle/depositing end on a substantially planar region matching at least with the target support (printing platform/support), the target support being integral with or included in the base portion.

The bioreactor can advantageously serve directly for maturation, cell culture or the like, without transferring the biological print, and possibly without any change of geometry/configuration at the level of the base portion. The driving system may simply be disconnected, if not used again in a further printing operation. After printing of the tissue or pattern, as programmed according to indications of a control unit associated with the driving system that moves the printing part (part including the adapter and the depositing end), the inlet port may be used to introduce nutriments, fluid and any required products for growth/maturation. At least one fluid outlet port is provided to evacuate waste or the like. the inlet and/or outlet port may be a connection port, forming a barbed nozzle. They are suitable for aseptic fluid connection with a flexible hose.

Near the base portion the side wall of the bioreactor may be relatively wide/comparatively wider than the bioreactor top opening or adapter flange. The side wall cross-section, of annular shape around a central axis, may vary. Whatever the way the side wall cross section varies (progressively decreasing or increasing, with a wavy profile or not), the side wall has a longitudinal extension, upwardly in an extended configuration so that the side wall lower annular end extends at the opposite from the upper end of the side wall.

The bioreactor may be provided with a plurality of connectors, including the connection port(s), the adapter allowing connection of the driving system outside the sealed enclosure, possibly port(s) for one or more sensors may be aseptic connections. In some options, a single interface connector may be provided, including several connections amongst such aseptic connections. At least one connection and disconnection may involve a clamp system, each associated a port(s). The adapter includes means for rigidly securing position of the needle forming the depositing end, so that relative position between the needle end and the adapter remains unchanged.

Thanks to such assembling of the sealed enclosure, the target support and the printing needle, nozzle or similar depositing end (printhead insertion part) may be provided in the interior volume, without any additional bulky embedded part of the printing system (for instance without any reservoir, driving system or the like being present in the bioreactor). In particular, the adapter can move with the flexible portion(s) of the sealed enclosure: such flexible portion follows movement of the adapter, thus moving together with the adapter, without any driving part (driving movement of the adapter) present in the interior volume. The top portion may directly cover/delimit the interior volume, being formed as a bioreactor top end at the opposite from the base portion.

When assembling the side wall, following options may be used:

-   the side wall has at least one welding area to join two edges of corresponding size, provided in at least one flexible sheet, so that a welded seam is obtained in the side wall;
- base material of the side wall is transparent and/or any part of the side wall is made of transparent material.
- the side wall surrounds the target support, which extends inside the sealed enclosure;
- the side wall has a lower end, of annular shape, directly connected to the base portion, using an annular flange or frame already provided in the side wall before connection to the base portion.
- the side wall has a lower end included in flexible sheet(s) of plastic material, this lower end being attached at a sealing area, of annular shape, to peripheral connection member provided in the base portion.
- after connecting the base portion to the side wall, a containing part is defined with at least two connection ports already provided in the containing part.
- each connection port may include a nozzle or similar fluid connector, preferably protruding outwardly, for instance protruding outwardly with respect to a circumference of the containing part.
- the method comprises forming at least one amongst the fluid inlet port and the fluid outlet port on the containing part.
- after connecting the base portion to the side wall, a containing part is defined with at least one fixation member/means protruding radially outward from a peripheral edge of the base portion, such fixation member allowing stabilizing the bioreactor when secured to a support or to a holding and retaining structure/device.
- the containing part is intended to be hermetically fastened to a covering part to form the sealed enclosure, which is sterilizable; a sterilization may be performed to have the sealed enclosure sterilized; in a variant, sterilization of the containing part and the covering part is performed when such parts are not assembled together.

According to a particular, the side wall and the base portion define a single containing part of the bioreactor for receiving biopharmaceutical product (during maturation of tissue print or cell culture). Optionally, the wall members of the bioreactor may each form/include a respective outer surface of the sealed enclosure, which is an outer surface of the bioreactor in absence of any additional bioreactor containing part surrounding the enclosure.
The base portion, the side wall and the top portion may be assembled at respective junction edges, in order to form an external wall of the bioreactor, to obtain the interior volume, preferably without any partitioning wall inside the interior volume.
Optionally, the top portion may be connected to or included in a piece involved in forming the annular side wall. For instance, the top portion may be a flap or extension of at least one flexible film already forming the side wall.
The base portion may be more rigid than a flexible portion of the bioreactor, which allows the adapter to be inserted in a lower compartment of the interior volume. Besides, the target support may be an integral part of the base portion, forming a rigid portion. Alternatively, the base portion is formed as a lower wall that is at least partly flexible, optionally made of a flexible film. Stabilizing tabs or fixation means may be provided to maintain the base portion (when it is flexible or when including a flexible region).
According to one embodiment, the at least one sealing operation is performed before any connection of the adapter with a driving system (including a terminal arm, for instance), preferably before any connection between the adapter and a cartridge or reservoir containing the biological material.
The bioreactor may be a single-use container, typically sterilized, which is connected to a printing unit (using a connection selectively joined to the adapter, for instance with a fastening region arranged outside the sealed enclosure). The adapter may protrude externally (for instance axially, away from the base portion) with respect to an outer surface of the sealed enclosure.
A cartridge or reservoir part of the printing unit may be removably connected to the adapter to allow the biological material to flow in a needle including the depositing end and crossing a central region of the adapter. The needle is in fluid communication with the reservoir and extends below the reservoir. The adapter may extend entirely below the reservoir part (of higher cross section that the needle/depositing end) part or may surround such reservoir part at an annular area when the driving system is secured to the cartridge/reservoir part at an external region thereof beyond the adapter and the top portion.
In some options, the assembling is adapted for allowing multiple cycles of bioprinting and cell culture. The bioreactor may include plastic material that is transparent, in order to facilitate checking progression of the bioprocess. Optionally, the top portion is provided with the adapter forming a first adapter, while a second adapter is also coupled onto a wall member that may be the top portion. This allows using two needles or similar depositing ends, for deposition of different material. In some options, collagen, another compound that is soluble after hydrolysis, a water-soluble compound may be an exemplary material deposited by a needle or similar depositing end associated to the second adapter. A combination of such materials may be used.
In an embodiment, a common adapter may cover two apertures provided in a same sheet part or similar flexible part. With such configuration, two depositing ends may be controlled by a same external driving system selectively coupled, directly or indirectly, to an outer connecting part of the adapter. In a variant using one or two septum parts, the two apertures may correspond to the pierced areas. Such septum parts may optionally be provided in a same adapter having an annular periphery for connection to the sheet part or similar flexible part. Alternatively, the two septum parts may be included in a same adapter forming a single flange or similar annular fastening member.

Before the sealing operation, a storage member may be provided, allowing storage of a depositing end. When several kinds of material have to be deposited, either there is a common needle adapted to deposit materials fed from different reservoir parts, or different printing needles (or similar depositing ends) are provided. When using two separate cartridges for feeding two separate needles both coupled to the top portion, two driving systems may optionally be coupled to the respective adapters, after achieving the sealed enclosure.

Before the sealing operation(s), the base portion may be formed as a rigid structure, suitable for keeping the printed tissue (obtained from the bioprinting). Possibly, the target support is formed by such rigid structure that extends parallel and/or along the base portion, using one or more securing members to have such structure rigidly secured to the lowermost layer of the sealed enclosure.

For instance, an additional layer of material distinct/different from material of the base portion may be secured to an inner surface of the base portion. Such additional layer may be rigid to form a substrate, optionally under form of a porous material (like fleece or membrane) and a welding may be used for immobilizing such layer. The base portion may also be a support for a plastic wall or similar additional layer printed (using 3D printing) or overmolded on the inner face of the base portion. Such additional wall part, housed in the interior volume or forming an inner face of the sealed enclosure, may be configurable depending on the tissue to be printed. In some options, the top portion is formed as an apex region of a dome-shaped flexible part, with the dome-shaped part (of half-spheroid shape for instance) including all or part of the side wall. Such shape is obtained in an expanded state of the flexible part, with a lower annular end of the flexible part secured to the base portion. The side wall thus may taper, upwardly, from the base portion or at least from a side wall region arranged adjacent/above the base portion.

The top portion and/or the side wall may include a stretchable flexible material, possibly silicon material. Of course, the sheet material for forming the stretchable part (including side wall may be in a flat) in an initial configuration before an expansion step, using injection of inert gas (typically injected from the bottom side of the sealed enclosure).

In some options, a treatment (for instance a laser treatment) may be provided to change physical and/or chemical characteristics of the inner surface of the base portion. Such treatment may be adapted for a rigid or flexible material forming the base portion.

In variants or in addition, a recess is formed to be the printing area/target area, as a cavity inside the base portion of the sealed enclosure; such recess may be obtained by thermoforming of a thin plastic sheet, or via manufacturing of an injected or printed rigid plate (by 3D printing for instance).

According to some options, integration of the adapter may involve one or more of the following particulars:

- the adapter is positioned distally from the side wall.
- the method comprises: forming the top portion using at least one sheet of film (possibly plastic film) or of a silicon material; and annularly sealing an annular outer edge of the top portion on a side wall region, preferably on an upper end of the side wall.
- the annular outer edge may be cut and/or sized, so that a deformable surface of the top portion is delimited by the outer edge and the interior volume is variable in the upstanding configuration as a function of a conformation of the top portion.
- the adapter may be surrounded by a sheet part forming a continuously annular surface that deforms.
- the surface delimited by the outer edge deforms without change in the area (deformation at constant surface area); such excess in surface allows the conformation of the top portion to be modified, for following movement of the adapter when driven externally by a driving system that extends outside the interior volume.
- the method comprises maintaining an upper end of the side wall in an annular conformation by spacing means directly coupled to a flexible portion of the sealed enclosure which surrounds the adapter, the flexible portion forming all or part of the top portion.
- the adapter is configured as a rigid connectable end part or extension of a driving system arranged outside the bioreactor, whereby the flexible portion can be displaced and follow movement of the adapter when the bioprinting with delivery of the biological material on the target support is performed.
- the adapter is placed to extend distally from a containing part of the enclosure/bioreactor, the containing part including the base portion and the side wall.
- the annular conformation of the side wall upper end is maintained by a rigid structure forming part of the side wall and/or by pulling two opposite portions of the side wall radially outwards (such arrangement allows maintaining a minimal interspace between the two opposite portions).

According to a particular feature, a film forming a sheet of the enclosure, optionally adapted to surround the adapter with a continuous annular leak tight contact, may have a thickness superior or equal to 100 $\mu$m. For instance, each film/sheet in the enclosure may have a thickness between 150 $\mu$m and 450 $\mu$m.
Alternatively, a thicker film or sheet (at least in a non-stretched state of such sheet) may be used. For instance, a silicon sheet/film of about 1 or 2 mm may be used. When such kind of stretchable sheet is used to form the side wall and/or the top portion, such thickness is defined before being stretched. A dome-shaped or similar expanded spheroid shape may be obtained, possibly starting from a flat sheet. Alternatively, the dome shape may be obtained due to a preforming step, using silicon material for instance.

According to an option for forming a flexible sealed enclosure, four wall members may be sealed together, with two of the wall members being interspaced gussets or bellows. Each of the films constituting the first wall member, the second wall member, the first gusset and the second gusset, respectively, has locally along the welds a thickness that is not less than the average thickness of the films.

The respective films may be each composed of at least three non-metallic plastic layers. Besides, the films may be transparent or translucent.

When forming a deformable surface, optionally away from the base portion, the deformable surface may be sized, so that in the upstanding configuration the top portion is movable from an intermediary folded state, which is preferably a flat state, to:

- an outwardly orientated expanded state that increases the interior volume, with the top portion being dome-shaped so that the adapter extends above the side wall in said upstanding configuration, and
- an inwardly bent state that reduces the interior volume, with the top portion forming a bioreactor recess for passage of a driving system intended to be connected to the adapter, while the adapter is surrounded by the side wall.

Deformability of the surface at the top portion may be due to surface area superior to a corresponding planar surface area/section delimited by an upper annular end of the side wall in the upstanding configuration.

The method may comprise varying capacity of the interior volume, due to movement of the top portion toward a position (possibly a lower position), in which the top portion is collapsed and adapted to be downwardly pressed by the adapter when the adapter is driven from outside by a driving system. The lowering of the enclosure capacity may be actuated by moving the driving system, after a connection step to make the adapter as movable (having same movement) as an end part/extension of the driving system.

In some options, before the bioprinting and/or during the bioprinting, the method comprises evacuating gas contained in the interior volume, possibly via an inlet port and/or an outlet port associated to the enclosure.

Gas evacuation and displacement of the adapter toward a lower position, with the top portion collapsed, may occur simultaneously in particular embodiments.

Whatever the way the interior volume is modified, a collapsed state of the top portion may be obtained, preferably while the bioreactor is still in an upstanding configuration. This allows the adapter to be moved, preferably by driving a robotized arm arranged outside the bioreactor, due to flexibility of a plastic material forming the top portion.

In some options, the method comprises varying a pressurizing level in the interior volume, in order to move the top portion between a collapsed state and an expanded state.

In some options, the outer edge may be polygonal. The method may comprise annularly sealing the outer polygonal edge to the side wall, for instance at an upper end thereof, using a covering part, the upper end (of annular shape) being preferably also polygonal to match with shape and size of the polygonal outer edge. Surface of the top portion, delimited by the polygonal outer edge, may be sized in a sizing step (for instance, using a cutting step) so that such top portion surface is superior to a corresponding area delimited by the upper end of the side wall (transverse section area delimited by the annular upper end).

According to an option, the forming of the top portion (using at least one sheet of plastic film) includes sizing and/or cutting a rounded peripheral outer edge, preferably continuously rounded and/or of generally circular shape. The upper end may also be rounded to match with shape and size of the outer edge.

In some options, the method comprises attaching a first elastic return member to the side wall and/or to the top portion, the first elastic return member being:

- involved in maintaining the annular conformation of the upper annular end, and
- secured to an external stationary structure provided around the bioreactor.

The adapter may be displaceable along one direction amongst the at least two transverse directions, to move away from the first elastic return member up to an offset position, in which the first elastic return member is extended to modify the annular conformation and locally narrow a perimeter of the upper end of the side wall. In some variants, no elastic return member is provided outside the enclosure and deformation is allowed in intermediate side wall regions provided between the fastening regions for connection to the external structure.

According to a particular feature, the method comprises providing at least one annular hinge in the top portion or along an annular upper end of the side wall to hinge the top portion. According to an option, a structure, rigid or provided with biasing means, is provided around the side wall, to retain the side wall such that this side wall extends distally around the target support. Accordingly, the bioreactor remains in the upstanding configuration, the structure with associated side wall links preventing contact between the target support and the side wall. The adapter may be moved in a region (inside the interior volume) surrounded by the rigid structure.

According to an embodiment, the method comprises one or more of the following features:

- a sealing/fastening of a number of flexible panels to each other is performed, such that when the bioreactor is in the upstanding configuration, they form at least the top portion and the side wall.
- in the base portion, at least a part thereof is made flexible, and an outer margin portion of the base portion is provided with radially protruding external extensions adapted for holding the base portion substantially planar or flat, along a base plane.

In a variant, the base portion may be a rigid plate.

According to options to perform the sealing operation to delimit the interior volume, the method may comprise:

- before the sealing operation to interconnect the base portion and the top portion, fabricating and/or mounting a receiving surface on an inner face of the base portion, which delimits the interior volume, in order to define all or part of the target support.
- providing a surface treatment at the target support.
- selectively use a peripheral margin of the base portion, around the receiving surface, for fastening with a complementary part, in order to obtain the sealed enclosure.

- the complementary part includes the top portion and has flexible material to delimit the interior volume from above.
- the complementary part is preassembled before connection to the base portion.

When the complementary part (forming a covering part) is preassembled, the adapter may be coupled to the top portion and arranged to protrude externally with respect to an outer surface of the top portion, before the fastening to the base portion. The protruding part of the adapter may include an outer connecting part, optionally forming a male part that is connectable to a female part provided in the driving system or vice versa (female part at the outer connecting part of the adapter).

According to an embodiment, the adapter, which is preferably a needle holder, is permanently connected with or to a base material of a flexible plastic sheet forming all or part of the bioreactor top portion.

the coupling of the adapter may comprise: pinching an annular edge of the top portion between two rigid rings of the adapter; or welding the annular edge of the top portion to a rigid peripheral part of the adapter.

The adapter and/or a printing platform forming the target support may be obtained by a three-dimensional (3D) printing technique from at least one printable material, preferably with the printable material and/or the base material being heated during printing thereby, so that the printed adapter (respectively the printing platform) is connected to the base material.

When all or part of the side wall is flexible, the side wall may be obtained by welding at least two pieces, at least one of which is made of a plastic film, preferably a multilayer film. Possibly, such at least one piece is provided with the fluid inlet port and/or the fluid outlet port. In some options, one or more gusseted portions are provided to form all or part of the side wall, the one or more gusseted portions being each welded or leak tight fastened to the base portion, preferably welded in a folded state to the base portion. The base portion may be also flexible, entirely or partly.

In some embodiments, the method includes a spacing step for spacing two opposite portions of the side wall, which is of annular shape in the upstanding configuration of the bioreactor, by spacing means, preferably under the form of stiffening means or retaining means. The spacing means may prevent the two opposite portions of the side wall from moving inwardly, toward one another.

In some options, the fluid inlet port and/or the fluid outlet port are provided in such opposite portions of the side wall.

Optionally, the method comprises stiffening an annular junction between the side wall and the top portion, the side wall and the top portion being made of flexible material, preferably of same multilayer film material.

Optionally, all or part of the top portion is made of rigid material and is configured to be movable due to flexibility of the side wall. When producing such arrangement to form the sealed enclosure, the method further may comprise a spacing step for spacing the top portion from the base portion, for instance using return members (possibly having tensioning effect), biasing members or similar holders, made separate from the side wall. In some embodiments, the return members/holders are coupled to the top portion and are configured to exert an upwardly biasing force, whereby the top portion is by default spaced from a predetermined distance from the base portion. After the bioprinting, such return members are of interest to ensure the side wall of the bioreactor is not remaining in a collapsed state.

When the top portion is made flexible, preferably made of one or more flexible film(s), the adapter is rendered displaceable with respect to the base portion. The adapter may be displaced between:

- a first position, distal from the base portion, in which the adapter is surrounded by a flexible part of the top portion that tapers upwardly or is dome-shaped and protrudes upwardly,
- and a plurality of second positions, in which the adapter extends proximal relative to the base portion with the flexible part folded downwardly (forming a, outer recess that opens upwardly in the enclosure).

In one particular embodiment, the method comprises varying a pressurizing level in the interior volume, in order to move the top portion between a collapsed state and an expanded state, the expanded state allowing the adapter to be moved above the target support, preferably by driving a robotized arm arranged outside the bioreactor, due to flexibility of a plastic material forming the top portion.

A pressure action may be applied, from above on the top portion in the expanded state, to have the adapter and the depositing end in a bioprinting position.

It is permitted to return to the collapsed state after the bioprinting, possibly after displacing the depositing end away from the target support and/or removing the depositing end. The interior volume can thus be reduced. Collapsed state can just mean that the top portion is closer from the base portion, not necessarily a total collapse of the flexible wall parts.

According to another particular embodiment, the method comprises connection steps for fastening the side wall at respective opposite axial ends that may include each a rigid disc, at least one rigid ring or similar rigid members. The fastening of the side wall may comprise:

- a first connection step for connecting the side wall, which includes or is a rigid member of generally annular shape, to the base portion; and
- a second connection step for connecting the side wall to the top portion that is made of at least one flexible plastic sheet.

Optionally, the second connection may be obtained before or after the first connection, or such connections may be obtained simultaneously.

When the base portion is rigid, at least a part of the side wall may also be rigid, so that the containing part of the enclosure is essentially/totally rigid. In a variant, the base portion is at least partly flexible, for instance made of a plastic film, and welded to an annular lower end of the rigid side wall.

The method optionally comprises severing an uppermost part of the top portion, after the bioprinting and after welding or pinching two opposite portions of the top portion at a welding area, in order to reduce the interior volume, the severed uppermost part entirely extending above the welding area.

According to a particular embodiment using a rigid coupler, at the top portion or adjacent to the top portion, the method comprises:

- at the opposite from the base portion, hermetically fastening a rigid coupler of annular shape to a flexible sheet of material adapted to delimit the interior volume from above and optionally adapted to form one or more flaps belonging to the side wall (the side wall being made of a plurality of flexible sheets);
- sealing a sheet assembly to the rigid coupler to cover an opening of the rigid coupler, the sheet assembly having an outer edge or flange directly fastened to the rigid coupler;
- sizing a top covering surface of the sheet assembly which extends above the rigid coupler, so that said top covering surface is adapted to be folded inwardly and outwardly, respectively, in relation to the rigid coupler, whereby the adapter can move along the at least two transverse directions below the rigid coupler without any driving part contained in the interior volume. The top portion may be formed by the flexible sheet, delimiting the opening matching with the rigid coupler (and corresponding opening), and the sheet assembly. The sheet assembly is made separate from the flexible sheet.

Optionally, the method comprises connecting a mobile member/stirring inner member of a stirring apparatus to the containing part, so that the mobile member extends inside the interior volume after forming the sealed enclosure.

It is understood a single interior volume may be delimited by the sealed enclosure. When a stirring/mobile member is provided, movement of such member may directly participate to homogenize fluid circulating at/along the target support (and associated printed tissue).

When performing the bioprinting, in the upstanding configuration of the bioreactor, the top portion may be conformed as an upwardly protruding protrusion in an expanded state thereof, possibly with a 3D shape forming an added volume of the interior volume. Optionally, the top portion may be dome-shaped due to a surplus of area as compared to the upper open area formed at the upper annular end of the side wall. Such upper annular end can remain substantially stationary when performing the bioprinting.

of course, the top portion may have many different conformations due to flexibility, possibly with an assembling between heterogeneous parts.

During the assembling, the top portion may be provided as a folded or gusseted part, or similar structure with an excess of film material used to obtain folds, at least when the top portion extends substantially parallel to the base portion (flat portion), in the upstanding configuration of the bioreactor (with the side wall substantially expanded for instance).

Practically, in such configuration, a developed length of the top portion, measured between two opposite ends of the top portion (with the adapter provided between such two ends) may be significantly superior to a maximum transverse characteristic size, possibly a diameter, of the annular upper end of the side wall.

The method comprises a connection to fasten the adapter to printer unit without inserting any hinged movable member of the driving system of the printer unit inside the enclosure. The method may also comprise locking such connection, possibly with an unlocking action permitted to separate the driving system without displacing the biological material depositing end relative to the adapter.

Such connection may involve an outer connecting part of the adapter that extends outside the enclosure.

The side wall and the top portion may be formed as a single piece of flexible plastic material, transparent for instance (possibly including silicone), in some embodiments. A dome shape may be provided for such piece. In variants, the side wall and the top portion are made of assembled sheet parts, flexible, forming a dome-shaped assembly. The flexible part of the enclosure may consist of or include the dome-shaped assembly.

In some options, the enclosure comprises a body part and a sheet assembly provided with the depositing end, the sheet assembly being connected to the side wall via a shoulder part of enclosure.

According to another aspect, embodiments provide a bioreactor for successively bioprinting a biological material and allow a maturation and/or cell culture phase based on the printed biological material (tissue), the bioreactor comprising:

- a sealed enclosure that includes a containing part provided with a base portion intended to form all or part of a lower end of the sealed enclosure, and a covering part, preferably including a top portion, to which a biological material depositing end is secured via an interface to protrude inwardly in an interior volume of the bioreactor, the interior volume being delimited by the containing portion and the covering portion, the base portion being provided with a target support;
- a flexible part made of one or more plastic sheets, adapted to follow a movement of the interface in relation to the base portion, in order to locally reduce the interior volume of the sealed enclosure;
- an upper end of the enclosure provided with an aperture and including a plug member adapted to plug the aperture and move in relation to the base portion, the plug member being included in the covering part to form all or part of the interface; and wherein the plug member, which is suitable to bear a needle or nozzle forming the depositing end, is integrated in the sealed enclosure to (functionally) interact with the flexible part so that the plug member is movable together with the depositing end along at least two transverse directions inside the interior volume, during a bioprinting phase to deposit the biological material on the target support.

In some options, silicon material or elastomer material may be used, preferably transparent. More generally, a flexible material having a shore hardness between 0.05 or 0.1 and 5, preferably transparent.

In some options, the sealed enclosure is provided with another (second) flexible part, which may function as pressure-compensation member. Such pressure-compensation member is configured to deform and expand with the sealed enclosure, for instance in response to a downward simultaneous displacement of the plug member and the (first) flexible part from a distal position to proximal position relative to the target support.

The bioreactor may include one or two connection ports to form a fluid inlet port and/or a fluid outlet port, adapted to be open for direct fluidic communication of an outer circuit with the interior volume after the bioprinting, so that the bioreactor is usable for a culture and/or maturation phase of the printed material/tissue inside the interior volume, after the bioprinting.

After the bioprinting, the bioreactor with the sealed enclosure is adapted to remain in a closed state, possibly without any uncontrolled entry of fluid. Of course, at least one connection port is used, for instance in a phase for introduction of the culture media (to be circulated and/or mixed) into the bioreactor, such phase being controlled by the operator, possibly using a controlling unit associated with sensors (temperature sensor, gas sensor, pH sensor are exemplary sensors that can be embedded in the bioreactor).

The adapter may define an annular sealing contact area of circular shape. In some options, the adapter extends distally from a containing part of the bioreactor which includes the base portion and the side wall, due to a stable conformation of the side wall and/or to at least one spacer included in or connected to the sealed enclosure, whereby the biological material depositing end, inserted through the aperture when secured to the adapter, can move while being prevented from laterally contacting the containing part of the sealed enclosure. A stable conformation may be due to a rigid material used to form and/or delimit the side wall. The adapter may be arranged centrally in the covering part connected to the side wall.

In some embodiment, spacing means, coupled to an annular peripheral region of the top portion, are configured to prevent the peripheral region of the top portion (4b) from moving toward the base portion. Possibly, the spacing means are movable in relation to two directions parallel to a bottom formed by the base portion, the spacing means being part of an external structure for positioning, retaining and/or holding the top portion.

In some embodiments, the adapter may be movable along three orthogonal directions due to flexibility of the top portion, possibly without modifying the shape and or a perimeter of the side wall (which is stiffened, made with rigid material at least in part, or held in position by an external retaining system).

In some embodiments, the bioreactor comprises a gas injection unit configured to inject gas, for instance inert gas, in the interior volume to obtain an expanding of at least one flexible sheet at the opposite from the base portion and maintain an annular peripheral region of the top portion above the annular side wall, while allowing displacement of the needle/depositing end or similar bioprinting head in the three directions.

The bioreactor is an assembly, preferably obtained by the above indicated method, such assembly comprising:

- a containing part (housing part) defining a longitudinal axis and having a longitudinal opening therethrough which forms the aperture, the containing part including the base portion and the side wall;
- the adapter, coupled to the enclosure away from the containing part, the adapter being suitable for adapting the depositing end, the adapter extending distally from the containing part and allowing the depositing end to be inserted through the longitudinal opening; and
- a seal member mounted within the longitudinal opening of the containing part, the seal member configured to receive the depositing end therethrough in substantial sealed relation therewith, the adapter being adapted to move in at least a radial direction, together with a flexible top portion of the enclosure, with respect to the longitudinal axis, the seal member maintains the substantial sealed relation between the containing part and a movable part including the flexible top portion.

The top portion may consist of plastic material that is flexible, having a given inner surface for delimiting the interior volume and deforming with the inner surface are remaining constant.

The bioreactor is a bioprocess bag provided with a number of flexible panels/wall member which are sealed to each other such that when the bioreactor is in the upstanding configuration, they form at least a top of the bag and a side surface (side surface of the side wall) of the bag.

At least one of the flexible panels/wall members may be forming the top portion and when the bioreactor is deformed, in the upstanding configuration, due to the adapter moving and downwardly pressing the top portion, the top portion will define a downwardly tapering projection of the bioreactor, bearing the depositing end, with the upper face of the bag having a depression.

Optionally, the depression opens only upwardly.

In a variant, the top portion is deformable so that the depression opens both upwardly and into the side wall.

In some embodiments, the top portion or another part of the sealed enclosure is provided with a venting port allowing compensating variation of volume inside the sealed enclosure.

The venting port may open into/communicates with another deformable container (typically also sealed) whose volume can increase when the interior volume of the bioreactor decreases and vice versa.

The bag/enclosure of the bioreactor may be obtained by sealing together flexible panels of polygonal shape, for instance of rectangular shape or hexagonal shape.

According to an embodiment, the base portion extends planar and may be provided with/attached to holding protruding members, protruding radially outward and configured to secure a given planar position of the base portion, which is a stationary position.

According to another aspect, a bioprinting system is provided, which comprises the bioreactor and a printer unit, wherein the printer unit comprises:

- a reservoir part filled with a raw content of biological material,
- a feed assembly, and
- a connecting part for coupling the printer unit to the bioreactor via the adapter that is bearing a needle provided with the biological material depositing end, so that the needle is a dispensing end part in a circuit of the printer unit,

wherein separating the connecting part from the bioreactor separates the needle and/or the adapter from the printer unit and enables the interior volume (V) to be kept hermetically closed.

In an embodiment, the bioprinting system comprises a driving system for providing a relative displacement between the bioprinting head and the target support, wherein the driving system and a reservoir of the feed assembly/cartridge extend entirely outside the bioreactor. Such driving system may comprise or consist of a robotic arm or a mechatronic actuator.

Optionally, the system comprises a support structure for maintaining the side wall and/or an upper annular end of the side wall stationary or keeping a substantially constant annular shape.

Besides, the system may comprise a printhead mount or depositing end coupled to a XYZ positioning device, here formed as a part of the driving system, so that the printhead mount directly drives displacement of the depositing end along three orthogonal directions.

BRIEF DESCRIPTION OF THE DRAWINGS

The figures of the drawings are now briefly described.

Fig. 1 is a schematic view of steps involved to produce a sealed container in accordance with an embodiment of the invention.

Fig. 2 is a perspective view of an exemplary printing system, using a bioreactor such as obtained via the steps of Fig. 1.

Fig. 3 is a diagram illustrating operations of forming and then using a bioreactor specially adapted for transferring living biological material inside a sealed enclosure without inserting the driving system in the interior volume thereof.

Fig. 4A and Fig. 4B are schematic longitudinal section view, showing a needle assembly fastened to a containing part of the bioreactor, respectively in an expanded configuration suitable for connection between an adapter and a cartridge movable thanks to a driving system such as illustrated in Fig 2, and in a retracted configuration suitable for the bioprinting, once an external upper end of the adapter has already been secured to the driving system.

Fig. 4C illustrates a sealed enclosure made with flexible sheets, with a holding and retaining structure allowing retaining the flexible containing part of the enclosure in an expanded state, with a wide access opening communicating with an upper part suitable for mounting a needle adapter.

Fig. 5A and 5B show two other embodiments of a bioreactor, having the adapter configured as a needle holder or septum, using different means to allow relative movement of the adapter with respect to a base portion, here a stationary base portion.

Fig. 6 depicts a non-limiting way of obtaining all or part of a sealed enclosure, using four complementary film parts that are adapted to be welded.

Fig. 7A schematically illustrates an assembling step with sealing operation, to obtain a sealed enclosure with another kind of movable top portion, here having flap portions or bent sides extending downwardly to form subparts of the side wall.

Fig. 7B illustrates a cut sheet of plastic film suitable to form the top portion, here with a polygonal shape in a flat configuration, before a coupling with the adapter, such top portion sheet part being adapted to be used in the kind of assembling showing in Fig. 7A.

Fig. 8A illustrates a needle assembly such as shown in Fig. 1, with a depositing end already secured to the adapter surrounded by the flexible material of the tapering or dome-shaped sheet part, with the depositing end protected by a sheath and by the sheet part that is provided with an end flange.

Fig. 8B illustrates a 3D-bag structure that can be obtained from several flexible sheets, for instance using the assembling of Fig. 6 or similar, with a top coupler of annular shape allowing fastening a movable a needle assembly at the opposite from the base portion.

Fig. 8C illustrates a needle assembly according to another embodiment, obtained from several flexible sheets with formation of gussets.

Fig. 8D illustrates another kind of needle assembly when integrated in the sealed enclosure, forming an annular welding separating the side wall from the top portion of the bioreactor.

Fig. 9 illustrates a connecting device provided outside the interior volume, involving an adapter outer connecting part and a corresponding connecting part formed on a printer cartridge.

Fig. 10 is a perspective view showing a printing system before lowering the adapter and a top portion of the sealed enclosure, to reach a lower level, in which a depositing end held via the adapter can deposit biological material on the target support.

Fig. 11A and 11B schematically illustrate, respectively a bioreactor and displaceable part of a bioreactor, provided with two depositing ends that may be associated to respective materials, in accordance with a particular embodiment.

Fig. 12 schematically illustrates a bioreactor provided with two depositing ends that may be associated to respective materials, in accordance with another particular embodiment.

Fig. 13 illustrates exemplary steps for having a mobility of the enclosure top portion, using a sheet of flexible material conformed as a dome, with the adapter (here not yet associated with a needle) arranged at a displaceable apex of the dome.

## MORE DETAILED DESCRIPTION

A detailed description of several embodiments of the invention is provided below, accompanied with examples and with reference to the drawings.

In the various figures, the same references are used to designate identical or similar elements. Some sizes may be exaggerated for the purpose of clarity. For instance, width or height of the containing part RC of some illustrated bioreactors may be exaggerated to improve illustration.

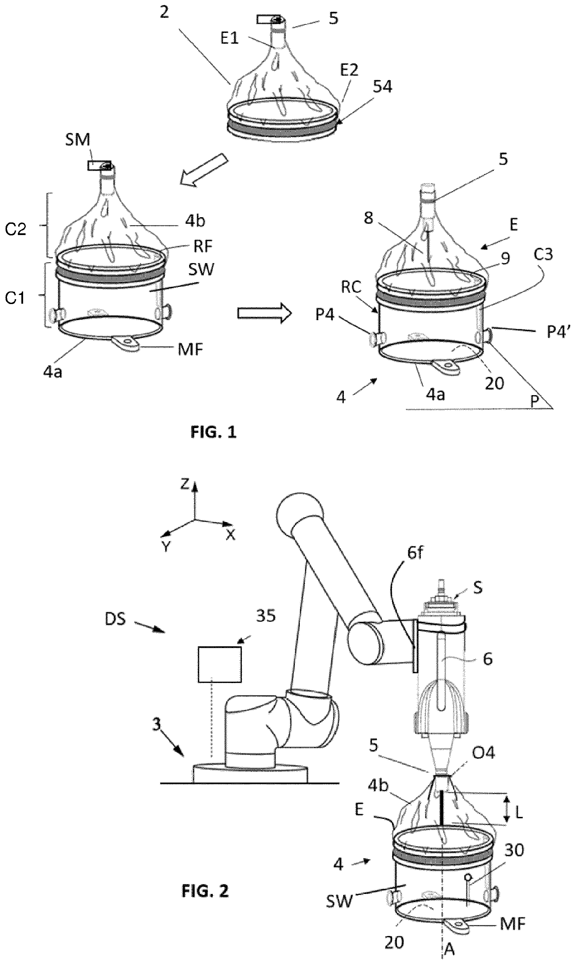

Referring to Fig. 1, it is shown exemplary assembling steps to form a single use sealed enclosure, adapted to be sterilized. A flexible part, made of one or more sheets 2 of plastic film may be used in fabrication of such enclosure, to allow the interior volume V to vary. The interior volume V may optionally correspond to juxtaposition of:

- a first compartment C1 defining a lower volume intended to be filled with a fluid during a bioprocessing phase,
- a second compartment C2, forming an upper volume with an annular space around a printing part that extends in the interior volume V, the second compartment C2 directly communicating with the first compartment C1.

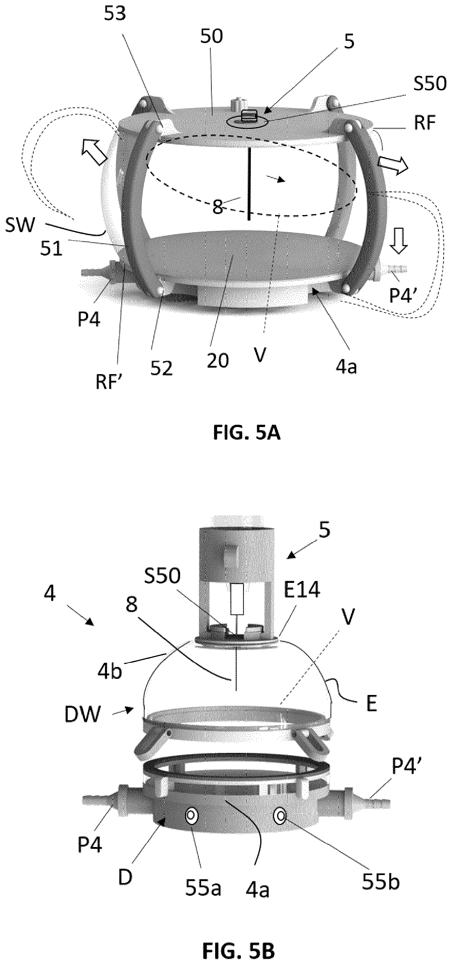

In a variant, the interior volume V may be not compartmented. For instance, the interior volume V may be such as illustrated in Fig. 5B and the side wall, secured to the base portion 4a is part of a dome shaped wall DW. In other words, the containing part RC may be laterally delimited by a side wall that tapers upwardly, possibly with a progressively curved/rounded profile. Assembling may be facilitated by using a simple sheet assembly 2 to form both the side wall and the upper end of the sealed enclosure E.

The sealed enclosure E may have a relatively small lower volume, inferior to 300 or 500 mL for instance, possibly with an average height or maximum height inferior or equal to 100 mm. Such sealed enclosure E is intended to serve for a bioprinting phase and then for a maturation phase. The containing part RC may be sized to receive less than 200 mL culture media, for instance about 100 mL culture media or less. The containing part RC may have a capacity as low as 10 or 25 mL, for instance.

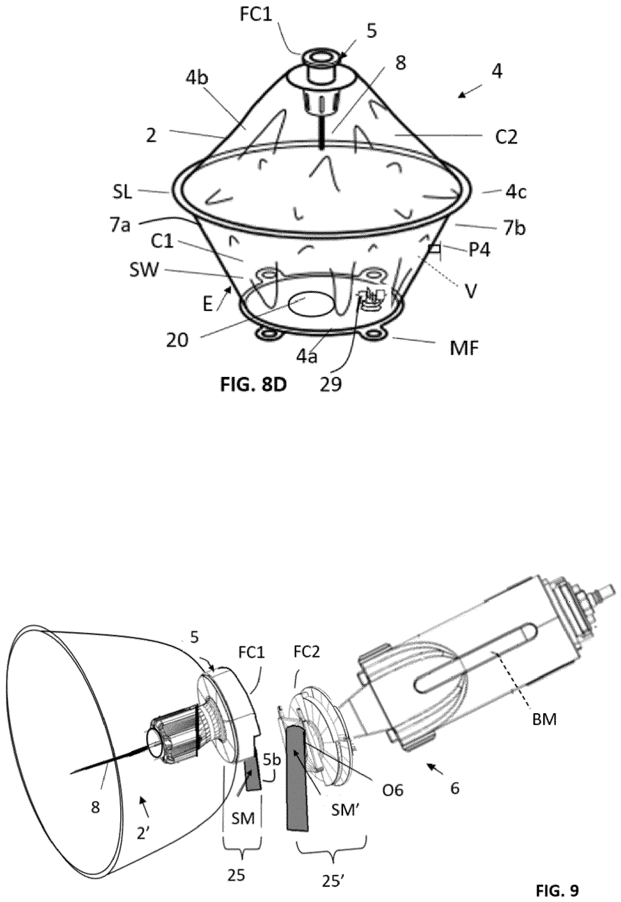

As illustrated in Fig. 2, the sealed enclosure E may be coupled, at a top thereof, with a driving system DS of a printer unit 3 having a feeding part filled with biological material. The sealed enclosure E is provided with an adapter 5, which is a connection member preassembled with the wall members of the bioreactor 4, for the attachment to a cartridge 6, 106 (see also Figs 9-12) or similar reservoir part containing biological materials BM, such as living cells. Such material may be viscous at least in the reservoir. Collagen or a hydrogel may also be included in such reservoir, with the cells or separately to be introduced. A depositing end 8, 108 with an opened free end is used to release the filament-like flow or similar flow (for instance a continuous flow or droplets) of biological material.

Whatever the design of cartridge 6, 106 and the way the adapter 5 is formed on the sealed enclosure E, the depositing end 8 may have a section calibrated to be able to deliver a continuous flow of bioink. A tapering profile may be provided. Size parameters at free end of the tapering depositing end 8 may be in the following range, for instance: 25-999 $\mu$m diameter (sub millimetric size) or with 1 mm diameter, possibly a diameter less than 3 mm. Other parameters may vary. The biological material can exhibit low viscosities (<10 mPa s). Cell densities may be also low (<$10^6$ cells ml$^{-1}$ for instance) in the material BM. In some variants, biomaterial depositing may also be performed spot by spot and/or micro-valves may be used just above the nozzle/depositing end.

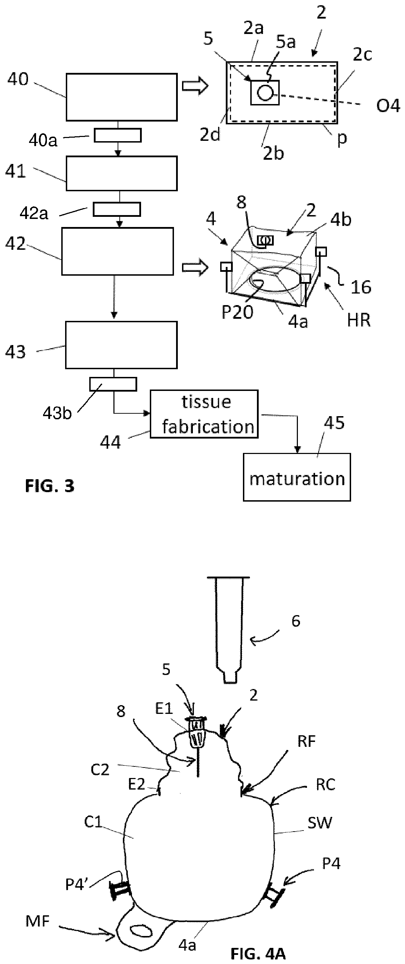

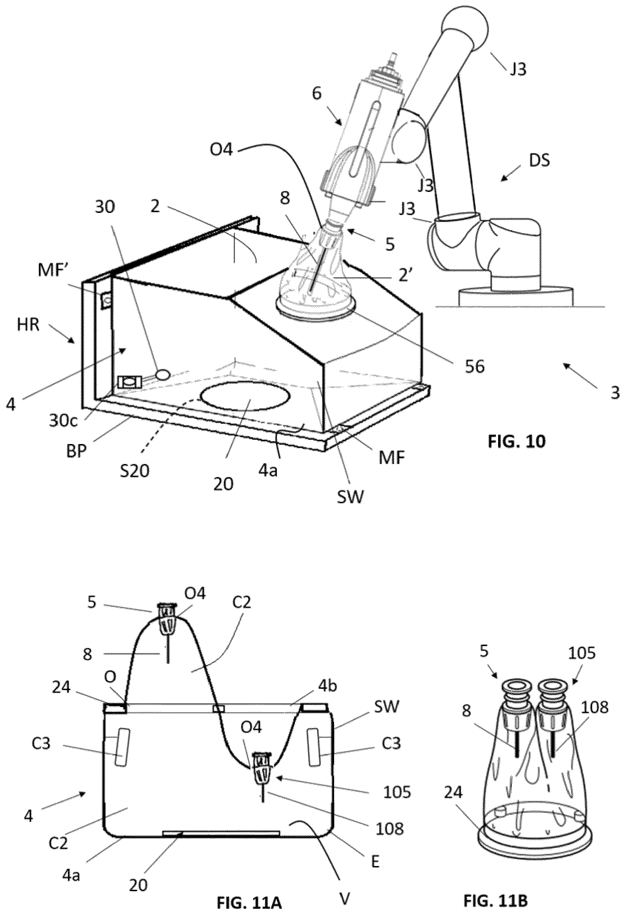

The driving system DS may be an articulated arm or any suitable a mechatronic assembly ensuring the relative displacement of a printing transfer area with respect to the target. In some options, two driving systems DS may be used to guide movement of two material depositing ends 8, 108, when constructing a structure (cube-shaped structure for instance) for cell growth or possibly when bioprinting a tissue T. Of course, the step 44 as illustrated in Fig. 3 is not necessarily a tissue fabrication and may depend on the material deposited on the target support 20. Referring to Figs 11-12, two depositing ends 8, 108 are shown, which may be received, simultaneously or not, in the first compartment C1. Fig. 11 shows a configuration with two adapters to allow a driving of the depositing ends 8, 108 by respective driving systems DS. Only one depositing end 8 or 108 may be displaced close to the target area 20 in this latter case. Each adapter 5, 105 may be surrounded by an annular edge E1 of a sheet part, delimiting the aperture O4, when the adapter is coupled to film sheet, away from the sheet outer side edges.

The bioreactor 4 has a base portion 4a, on which a target support 20 may be formed. A positioning relief 4h may be provided at the base portion 4a, for instance to interact with a complementary relief of a support part in contact with the enclosure from below. The base portion 4a may be of polygonal shape, preferably rectangular. Alternatively, the base portion may be continuously rounded, for instance of circular shape. The interior volume V is delimited laterally by a side wall SW, in an upstanding configuration of the bioreactor 4. Such interior volume V extends/is delimited between the base portion 4a and the top portion 4b, in an upstanding configuration of the bioreactor 4. An aperture O4 of the top portion 4b is plugged by a piece or assembly belonging to the adapter 5. When coupling (at a specific step 40) the adapter 5 to the sheet part 2 or to a rigid upper plate/member arranged opposite the target support 20, an annular sealing is obtained to prevent gas passing around the adapter 5. Step 40 may be performed before another attachment step to delimit the interior volume V. In some options, several adapters 5, 105 are arranged, typically at the top portion 4b and each in a movable part. Fig. 11 shows a top portion 4b that includes two flexible parts that can move in opposite directions. A stiffening may be provided around such flexible parts or the top portion 4b may be provided with a flange 24 or frame, delimiting the top access passages for communication with the compartment C1.

Besides, the adapter 5 may include at least one inner channel intended to be filled with the material flowing, downwardly for instance. A needle or similar depositing end 8 may extend the adapter 5 or be inserted therethrough, with a fastening that is rigid, preventing relative movement of the adapter in relation with the depositing end 8. The adapter 5, 105 may be a rigid plugging member that seals the corresponding aperture O4, after a heat welding operation or similar welding. The adapter 5, 105 may be a tri-clamp adapter, optionally suitable to clamp a needle or similar printhead, forming the depositing end 8, 108. The adapter 5, 105 may be a luer lock adapter, allowing insertion of the depositing end 8 in the interior volume V.

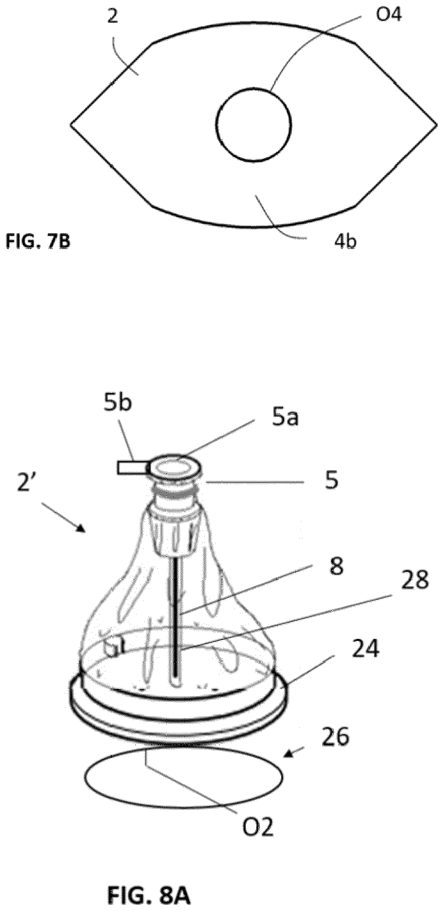

The adapter 5, 105 may be provided with any suitable outer connecting part, allowing the depositing end 8 to be fed with the biological material(s). In some options, the adapter 5 may be a septum member with the channel formed when piercing the septum S50 (see Figs 5A-5B). Also, the adapter 5 may be a sterile connector provided with a seal membrane SM (see Fig. 1 or Fig. 9), for instance a gas permeable membrane or a gas tight membrane. Such membrane SM is used as sterile barrier, covering an open end of the outer connecting part FC1 of the adapter 5. As shown in the exemplary adapter 5 of Fig. 8A and 9, a seal membrane SM may be provided with a disc-like cover part 5a (covering an upper end of the adapter 5) and a flexible pulling part or similar tab 5b having a base connected to the cover part 5a.

The side wall SW may be able to form a self-supporting structure or, alternatively may be coupled to a holding structure HR. While Fig. 1 shows a rigid (or at least rigid-in-part) containing part RC including the side wall SW of generally annular shape, the side wall can alternatively be made of flexible plastic material with an inner face adapted for contact with biopharmaceutical fluid. Fig. 4A illustrates an example with the containing part RC being flexible, possibly except the base portion 4a. In some options, a semi-rigid or rigid member, possibly a clamping member 54 or outer fastening ring or flange, may be provided at a fastening region RF between the flexible sheet part 2 forming the top portion 4b and the annular upper end of the containing part RC intended to be filled with biopharmaceutical fluid after the bioprinting step 44. A welding may be performed to ensure gas tightness at such fastening region RF.

A rigid or semi-rigid collar may be involved as an interface for sealing, for instance by welding, two respective annular ends distributed in the flexible sheet material of the side wall SW and the flexible sheet material of the top portion 4b. Such collar may be a rigid or semi rigid, forming a coupler already mounted on one amongst the top portion 4b and the side wall SW. Such coupler is formed with an associated annular sealing on a flexible sheet material at a peripheral area thereof, before the connection to form the annular fastening region RF. Also, the adapter 5 is already coupled to the top portion 4b.

More generally, the bioreactor 4 comprises, opposite the base portion 4a, a top portion 4b provided with the adapter 5 which carries or is traversed by the tool forming the printing transfer area. Such tool may be a needle, a nozzle or similar depositing end for depositing biological material in a bioprinting phase 44. As illustrated in Fig. 3, such bioprinting phase 44 can be performed after a preparing phase (40, 41, 42) in which the bioreactor 4 is assembled (typically preassembled and typically after a connecting step 43 to make the adapter 5 integral with/rigidly secured to a moving part of the printer unit 3. Such moving part may include the reservoir part or cartridge 6. The bioreactor 4 may be also sterilized, in a sterilization step 42a. The sterilization step 42a may occur before the bioprinting phase 44. Such sterilization step 42a may be performed after the connecting step 43 in some embodiments. Sterilization is of interest, to allow bioprinting and permit subsequent culture in a same chamber (in the interior volume V) in a sterile way. Possibly, a sterilization step 42a may be performed when assembling the parts at the connection step 43, or just before performing such connection.

The one or more sealing operations to obtain the sealed enclosure E may be performed before any connection between the adapter 5 and a cartridge 6 or reservoir containing the biological material BM. With such sealed state, the bioreactor 4 as initially produced may be initially conformed in a collapsed or substantially flat state of at least the top portion 4b, and with the side wall SW folded to limit height when the side wall is flexible. In some variants, the enclosure E is already produced in an expanded state, possibly with rigid members or the whole rigid side wall maintaining an axial spacing between the base portion 4a and the top portion 4b.

Whatever the way the enclosure E is produced/assembled, the bioreactor 4 is partly flexible to delimit the interior volume V, in particular with the top portion 4b being flexible and/or laterally displaceable.

When the side wall SW extends around a longitudinal axis A, with the bioreactor 4 upwardly extending in a stable configuration, the top portion 4b is suitable to move laterally and shift the aperture O4 radially in different directions. While some options provide the aperture O4 crossed by the longitudinal axis A, for instance in an upwardly expanded state of the top portion 4b, such aperture O4 may also be slightly shifted to be initially close to an outer edge E2 of the top portion 4b.

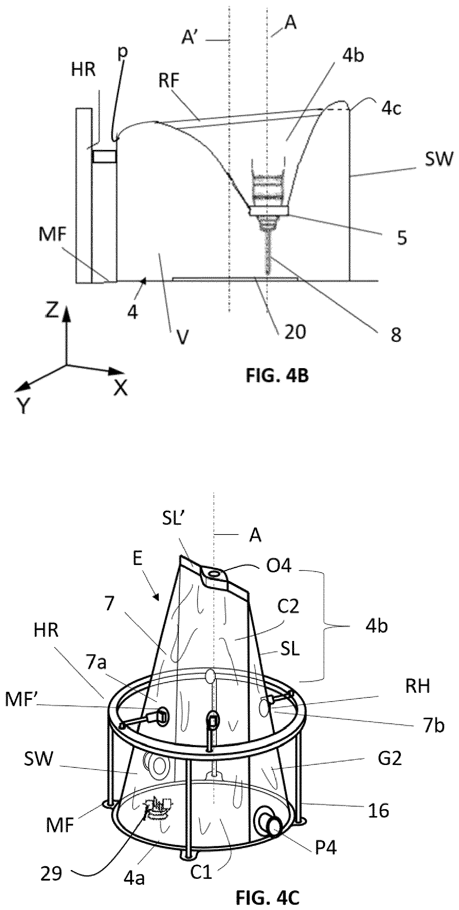

Considering the longitudinal axis A' of the side wall SW, the top portion 4b may be provided, at the aperture O4, with the adapter 5 coinciding or not with this axis A'. When displaced in transverse directions X or Y, an initially centered adapter 5 may be shifted laterally in any radial direction. Fig. 4B reflects a position of the adapter 5 when depositing a bio-ink/material with the adapter longitudinal axis A significantly shifted as compared to the longitudinal axis A' (here a central axis) of the side wall SW.

The target support 20, constituting a printing platform (substantially planar for instance), may extend transversally with respect to the longitudinal axis A that is typically a vertical axis. An adapter storage position may be reached after the bioprinting, which is:

- an uppermost position for the adapter 5 or similar raised position, so that the depositing end 8 located in the volume V is maintained away from the target support 20,
- or a laterally shifted position.

A connection with an external holder may be used to prevent downward movement of the adapter 5 toward the target support 20.

When laterally shifting position of the adapter 5, 105, a needle storing compartment C3 may be provided in the interior volume V. A purging of the needle may be performed when stored in such compartment C3, which may be provide with a bottom. A sheath 28, case or an annular retainer may be provided on inner side of the sealed enclosure E, with a retention part 9. The retention part 9 may be an integral part of the sealed enclosure, made of same material as a material used in a wall member of the containing part RC. In exemplary embodiments, the retention part 9 includes an upper abutment edge for contact with a bottom surface of the adapter 5. A suitable passage section is delimited by the upper retention part 9, allowing passage of the needle or similar depositing end 8 from above, until inserted and retained to prevent accidental displacement of the adapter with such end 8 in a central region of the interior volume, just above the target support 20 for the bioprinting.

In some options the depositing end 8, 108 can be coupled to a hose allowing purging of biological material or similar (which may include cells, extracellular matrix (ECM) components, growth factors and other biomaterials) before the bioprinting phase 44. A calibration step 43b may be performed after forming the sealed enclosure E (and after having the adapter and the depositing end associated with the printer unit 3), with a retrieval of an amount of biological material that is not used for the bioprinting on the target support 20. A collector may be embedded inside the bioreactor 4. For instance, the sheath 28 may include/form such collector for collecting an amount of material delivered from a needle, hose or similar depositing end 8, during the calibration step 43b.

Besides, a flow rate of the flowable biomaterial/bio-ink may be adjusted in some embodiments. A section of the nozzle, end hose or similar depositing end 8, 108 may be adapted for a given flow rate range or given flow rate value. Optionally, the depositing end 8, 108 may be adapted to continuously deposit the material according to a grid design, for instance with several loops, without interruption in the flow. A continuous filament thus may be deposited. Superimposed grids of such material may be printed in some options, for instance to promote cell colonization of the printed matrix using a bottom-up approach. The flow rate is calibrated at the calibration step 43b so that the depositing end 8 can deliver a filament of constant/substantially constant section.

For instance, an inner diameter of the depositing end 8, 108 is inferior or equal to 3 mm, for instance inferior or equal to 0.8 mm, and superior or equal to 25 $\mu$m. The cross-section at such depositing end 8, 108 for the flow of flowable material to be dispensed during the bioprinting phase 44 may be comprised between 200 and 800 $\mu$m. Additionally, or independently from that, cross-section of the needle or similar hollow depositing end 8, 108, as measured inside the bioreactor 4, may be much less (at least 10 less) than cross-section of any of the ports P4, P4' of the bioreactor 4.

When the adapter is a rigid part surrounded by a flexible sheet material, the adapter may include an annular ring or flange to which the edge E1 is secured. Such flange may have a diameter inferior to 30 or 40 mm, so that flexible material of the sheet part 2 or 2' is spaced from less than 20 mm from a longitudinal axis A of the adapter 5, 105. The side wall SW may remain spaced from at least 20 or 30 mm; possibly at least 50 mm from the longitudinal axis A, due to parts of the side wall SW that are retained or made rigid. A spacing is thus created, either at a particular step 44 prior to the bioprinting, possibly after the sealing operation at step 41 when the side wall SW is generally flexible.

The adapter 5, 105 may be designed with leaktight interaction members, arranged to define at least one continuous annular sealing contact. Such leaktight interaction members may be provided for accommodating/adapting the depositing end 8, 108. Typically, the leaktight interaction members may be in some options a perforable cap or a leaktight seal (septum S50, luer lock or the like).

In some options (including options compatible with absence of a retention part 9), the bioreactor 4 may have a flexible top portion 4b that is sufficiently long to allow, after the bioprinting phase 44, a welding at two complementary inner face portions of the top portion, before a cutting to remove the adapter 5 with the depositing end 8 (weld & cut intermediary step for reducing the volume V, before the bioprinting). More generally, the top portion 4b may be optionally configured to allow severing or removing at least a part thereof. For instance, an uppermost part of the top portion 4b is severed after the bioprinting and after welding two opposite portions of the top portion 4b at a welding area, the severed uppermost part entirely extending above the welding area.

Besides, the bioreactor 4 may be configured to facilitate retrieving the tissue after a maturation step 45, by a unlock device releasing access to the base portion 4a, for instance by allowing displacement/separation of the side wall SV above the base portion 4a. Alternatively, an annular cutting step may be performed to sever at least one amongst the side wall SW and the top portion 4b.

After fixing the containing part RC to the flexible/movable upper part, the sealed enclosure E is obtained. Referring to Fig. 4B, it can be seen that the coupler or similar part for obtaining the fastening region RF delimits a passageway for passage of the needle or similar depositing end 8 between the second/upper compartment C2 and the first/lower compartment C1, allowing the depositing end 8 to be inserted in such lower compartment C1 delimited by the side wall SW. Fig. 4B illustrates downward folding of the top portion 4b, which may at least in part extend below an annular upper end 4c of the side wall SW.

The bioreactor 4 is thus provided with a highly flexible part, here at the sheet part 2 that forms the top portion 4b. Such top portion 4b may have a convexity for the outer surface thereof (convex top portion) as view from outside the sealed enclosure E), which is changed to a concave conformation upon applying and maintaining a downward pressure or similar downwardly orientated force in a region adjacent to the aperture O4 of the top portion 4b, due to displacement of the adapter 5 when driven by the driving system DS. A movement along XYZ directions may be obtained when bioprinting a tissue by the depositing end 8, on the target support 20 that remains stationary with the base portion 4a. Such base portion 4a may be prevented to move, for instance using a holding and retaining structure HR that blocks the base portion 4a at tabs of similar fixing extensions MF integrally formed with the base portion 4a and/or with the side wall SW.

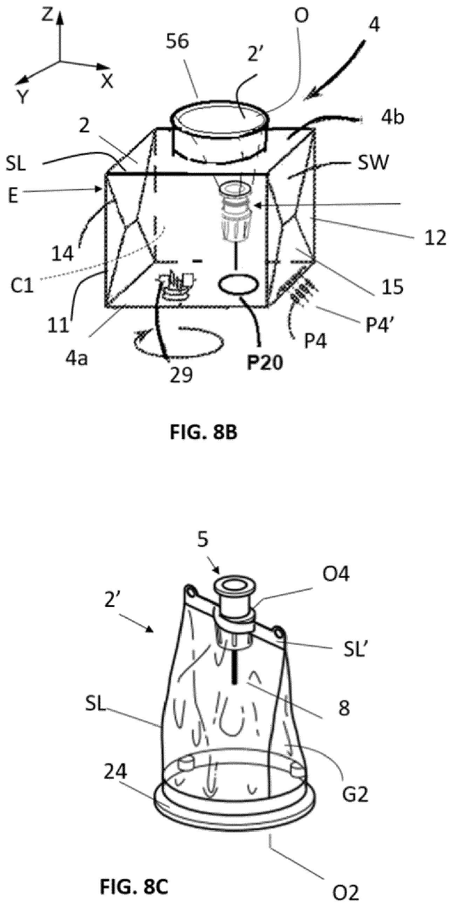

Such stationary state may be maintained, partly or entirely, during preparing phase to obtain the sealed enclosure, during the bioprinting step 44 and then optionally when performing a maturation phase, typically using one or more fluids, i.e. with biopharmaceutical fluid received in the containing part RC. A fluid movement or a stirring may be performed, while still maintaining the base portion 4a stationary, for instance using the holding and retaining structure HR and with an upper position of the adapter 5 not interfering with the bioprocess inside the bioreactor 4. Fig. 8B shows a non-limiting example for a stirring member 29, here arranged in the base portion 4a and laterally shifted with respect to the target support 20. Any suitable stirring member 29 may be mounted on the base portion 4a, before forming the fastening region RF with the flexible part of the sealed enclosure E, i.e. before installation of the covering part (2, 2').

Generally, in the instant disclosure, "biopharmaceutical fluid" is understood to mean a product resulting from biotechnology (culture media, cell cultures, buffer solutions, artificial nutrition liquids, blood products and derivatives of blood products) or a pharmaceutical product or similar intended for use in the medical field, for instance in regenerative medicine. Such a product may be in liquid/fluid form. Gas may also be produced during bioprocess and the sealed enclosure E may be gas tight. A sterile exhaust gas vent can be added in some options, for instance including a filter. Such vent may be provided as a function of the cell culture to be prepared in the bioreactor 4. Material(s) BM, M may be a minor part of the enclosure content when performing the bioprocess (culture/maturation), at least when beginning introduction of a culture media (with nutriments).

In some embodiments, the depositing end 8 may extend inside a sub volume delimited by the flexible part in an expanded state thereof. The containing part RC may define a nominal volume, which is a first volume of the sealed enclosure. The sub volume corresponds to a second volume that may vary. In expanded state of the flexible part, the following relation may be satisfied, for instance:

$$0.1 < V2/V1 < 1$$

where V1 designates the first volume (corresponding to the first compartment C1) and V2 designates the volume of the upper/second compartment C2 as obtained with the flexible part expanded to have the adapter extending entirely above the first compartment C1.

More generally, the interior volume V may vary. The depositing end 8 is provided in a needle or nozzle protruding in the interior volume V, in order to keep a determined length L (that may be superior or equal to 9 mm). This may be suitable to deposit bio-inks, typically viscous printing fluid droplets including living cells.

The side wall SW may be higher than length L, while a movable flexible assembly 2, 2' (forming all or part of the covering part) including the adapter 5 and connected to a top of the containing part RC may also have a height greater than length L, for instance with a movable assembly maximal height greater than 20 or 30 mm. Such height corresponds to height of the upwardly tapering sheet part, without taking into account the adapter 5, 105 that is typically rigid and protrudes axially outward.

**Assembly option with external holders**

The side wall SW may be flexible, rigid or partly flexible. Fig. 5A illustrates a non-limiting embodiment with the top portion 4b being a rigid member 50, here a rigid plate that is articulated and/or supported by partly flexible holders 51 (here with ability to be bent according to a bending, possibly without twist effect) that exert a return force toward a raised/upwardly extending configuration of the bioreactor. Flexibility of the side wall SW here compensates rigidity of the top portion 4b and the flexible holders may be arched with varying curvature. The side wall may be a pouch-like side wall SW, secured by welding or similar suitable sealing onto an annular peripheral region RF' of the base portion 4a. The side wall SW is thus maintained at its opposite ends, by the axial opposite portions 4a, 4b (with respective regions of fixation RF, RF'), knowing the top portion 4b is suspended due to the holders 51. Decreasing radius of curvature for at least some of the holders 51, here elongated holders of resilient material (silicon or similar for instance), occurs when a pressure is applied on the adapter 5 to reach a lower position for bioprinting by the depositing end 8. Hinge links 52, 53 are provided at the two opposite ends of each arm/holder 51, for respective connection:

-   to the base portion 4a, at lower end of the holder 51, via the lower pivot 52 ensuring the hinge link; and
-   to the top portion 4b, at an upper end of the holder, via the upper pivot 53 ensuring the hinge link.

Any kind of assembly with resilient holders, for instance with at least three or four holders, may be suitable to guide movement of the rigid member 50. With such configuration, the adapter 5 may be included in the plane of the rigid member 50, with a septum S50 arranged in a central area thereof. The needle/depositing end 8 can be inserted through a plasma septum or similar septum, without making the side wall SW collapsing, thanks to supports/holders.

In embodiments with a flexible side wall SW, the housing/enclosure E of the bioreactor 4 defines an interior volume whose delimitation is varying, with the base portion 4a forming a stationary part, possibly the sole stationary part.

When such side wall SW is made of a flexible plastic film/material, for instance silicon material, the side wall SW may expand from a relatively flat conformation to a deployed conformation. In both configurations, the side wall SW is part of the enclosure E which is annularly connected to the base portion 4a that remains flat or planar along a base plane P. Upstanding configuration of the bioreactor 4 may correspond to such deployed conformation. For example, polyethylene may be included as an inner layer of a monolayer or multilayer film included in the side wall SW and/or the top portion 4b. Alternatively or additionally, the side wall SW may be at least partially produced from silicon material(s), possibly for the wall parts intended to be in contact with the culture medium after the bioprinting.

When flexible, the side wall SW and/or the base portion 4a may be maintained (with a holding effect) to delimit a substantially constant volume for compartment C1, by the holding and retaining structure HR. Such holding effect may be obtained by exerting a pulling force radially outwards, using tabs or similar extension/fixation means MF provided laterally on the side wall SW. Additionally or alternatively, an upwardly orientated force may be applied to an upper end of the side wall SW.

Referring to Fig. 10, a holding and retaining structure HR is illustrated, including a L section for guiding position of the base portion 4a one the one hand, and position of at least one side of the side wall SW. The bottom of the structure HR may be a bottom plate at least partly extending below the base portion 4a, to maintain such base portion 4a substantially flat, along a base plane. The peripheral part of the base portion 4A may also be configured to remain stationary, for instance due to a rigid conception.

A substantially vertical rigid wall, transverse to the bottom, is here illustrated, allowing clipping or other fixture to tabs or similar fixation members MF' of the enclosure E, provided on the side wall SW, for instance away from access at the top portion for a depositing end 8. The fixation means MF arranged at or close to the base portion side may extend comparatively closer to the target support 20 (collection plate, planar surface or the like). Besides, whatever the way flexibility is provided in the sealed enclosure E to allow displacement of the adapter 5, the holding and retaining structure HR may have one or more guiding reliefs (protrusion or recess), optionally selectively arranged in a central region of the bottom plate BP. With a complementarity in shape or an abutment or friction effect, possibly with a rotational locking, a corresponding surface S20 of the base portion 4a may be secured to the bottom plate BP of the structure HR. More generally, the sealed enclosure E may lie on a lower part of a holding and retaining structure, possibly with a stretching effect, when the base portion 4a is not intrinsically rigid enough to remain planar. The inner face forming surface S20 may be planar and configured to have no wrinkle or relief.

The structure HR may optionally surround the side wall SW. Referring to Fig. 4C, the holding and retaining structure HR may be provided with a ring or similar surrounding part coupled to several attachment fixation members MF' of the enclosure E. Such surrounding part may be connected to raising rods, jambs or similar supporting members 16, distributed around the enclosure E. When gussets G2 are provided in the enclosure E, the structure HR allows the gussets G2 to remain substantially unfolded, thus with a spacing between, the two opposite main flexible sheets 7. Before the sealing step 41 forming the enclosure E, the fixation members MF' may be formed on respective sheets (7 and/or G2) that are then assembled at step 41. The fixation members MF' may be added away from the welding regions or similar sheet fastening regions.

Possibly, one or more elastic return members may be engaged for connecting the side wall SW of the enclosure E to a stationary part of the structure HR. This may allow slight deformation, for instance for compensating a small increase or decrease in pressure in the lower region of the interior volume V.

At least two, three or four fixation members MF' may be distributed in a same annular region of the enclosure E, possibly at same height level that delimits an upper end of the containing part RC or similar wide enclosure part used for maturation phase and delimiting the compartment C1. Due to retention effect at the lower fixation means MF, a lower part of the enclosure E is less deformable than an upper part. More generally, any suitable structure may be provided to have the enclosure more deformable in a top portion 4b thereof, as compared to a lower portion thereof including all or part of the side wall SW.

Referring to Fig. 4A to 4C, except at a holding region RH, in particular at the fixation members MF', or similar fixation region RF, the enclosure E may be:

- slightly movable in the side wall SW, between the base portion 4a and the region RH or RF, with limited local deformation; and
- highly movable beyond the region RH or RF (at top portion 4b and/or in a flexible sheet part 2, 2'), possibly with ability to fold the plastic material of enclosure E so that a rigid upper end of the enclosure may be displaced in the compartment C1.

In some options, the rigid end may include a transverse welding junction SL' for junction of film sheets and/or a top clamping member that remains outside the interior volume V of the bioreactor 4. A tightening system like a cable tie may be involved in some embodiments. The rigid end mays also be provided with the adapter 5 and an associated depositing end 8 (no needle illustrated in Fig. 4C). The rigid end may form the aperture O4, which is crossed by a longitudinal axis A of the adapter. In an uppermost extended configuration of the top portion 4b, such axis A may be a symmetry axis or a central axis of the side wall SW, possible a central axis of the sealed enclosure E.

In some options, the inner face of the respective wall members delimiting the interior volume V of the sealed enclosure E may be composed of a given plastic material. More generally, such wall members allow a change in conformation of the enclosure E, typically with a reduction of the volume when the depositing end 8, 108 is in position of transferring biological material on the target support 20, while the volume may be increased after the bioprinting step 44, in order to maintain the depositing end 8 away from the target support 20. Such change in conformation can be obtained by use of folds and/or reliefs, excess in surface formed in the top portion 4b, possibly with formation of one or more annular hinges around the aperture O4 of the top portion 4b.

Referring to Fig. 4A, the flexible part included in the top portion may be folded inwardly due to flexibility of film(s) forming at least the side wall SW and the top flexible sheet assembly 2. The lower compartment C1 may be similar to the case of Fig. 1 but with a flexibility in the side wall SW and/or use of same material to delimit the respective compartments C1, C2. In some variants, at least one hinge may be provided, preferably with each hinge being of annular shape. One or more external folds may be provided in the top portion 4b, even when the top portion is dome shaped as illustrated due to flexibility of the film material. Possibly, a stiffening may be provided in an annular region to have a predefined hinge effect and better control variation in the conformation of the top portion 4b. In any case, the top portion 4b may be well adapted to plunge in the compartment C1 with a high degree of freedom regarding movement of the adapter 5 (here along XYZ directions). The flexible sheet part 2 provided at the top of the bioreactor 4 may deform for constant surface area, due to change of conformation. Optionally, a wavy profile for the sheet material of the top portion 4b may be provided, so that several grooves (not shown), for instance continuous annular grooves or discontinuous grooves with an annular distribution of the groove portions.

The design of Fig. 4A is suitable for obtaining the top portion 4b and the side wall from one or more flexible sheets.

The sheet part 2 may have an outer edge E2, delimiting a periphery p of the top portion 4b, that remains substantially at same height level of the upstanding bioreactor 4 and conformation of the top portion 4b obtained in assembled state of the sheet part 2 varies to protrude either upwardly or outwardly, with respect the height level of the periphery p (without reducing or significantly reducing the compartment C1 for instance). The different parts of the sealed enclosure E may vary to obtain such ability for the region of the top portion 4b provided with the aperture O4 to be displaced inside the volume V with a variation of conformation for the upper compartment C2. Some examples for producing the bioreactor 4 and options for assembling the different parts will be described below.

**CONSTRUCTION OF THE SEALED ENCLOSURE**

Now referring to Fig. 3, it can be seen that the enclosure E may be obtained from different pieces, in order to delimit the interior volume V. The method for the assembling comprises, for instance:

- two preliminary steps, including delimiting a part 2 and/or 2' (covering part) intended to cover the containing part RC and providing an aperture O and/or O4 in such part;
- a first assembling, which includes a coupling step 40 for mounting the adapter 5 as a fitting inside the aperture O4; this step 40 may be performed after the two preliminary steps or may be obtained when conceiving the part, for instance if the adapter can be obtained directly when forming/molding the part 2 with the adapter 5 included/overmolded on the part 2;
- providing the side wall SW and the base portion 4a and optionally securing a printing platform on the inner face of the base portion 4a; the base portion 4a may optionally be made with at least one planar part, which is separate from the top portion.
- securing to the base portion 4a and/or to the side wall SW (for instance in a position adjacent to the base portion 4a), one or two connection ports P4, P4', with optionally a barbed male connector suitable for connection with a flexible hose.
- providing a peripheral area with tabs or similar fixation means MF extending outwardly, or including rigid material and/or holding parts in the side wall SW.
- forming 41 the sealed enclosure E, possibly with welding operations or similar gas tight connections at one or several annular junctions/overlapped segments (which may distributed in edges of a polygonal periphery for the base portion 4a and/or the top portion 4b).

A sterilization step may also be performed, for instance just after forming the sealed enclosure, to sterilize the enclosure E. While Fig. 3 illustrates a sterilization step 42a performed after connecting the adapter 5 the precise order of the steps may vary: of course, steps 43 and 42a may be performed before a step 42 for preparing a structure for maintaining the bioreactor 4. The sterilization step 42a may be performed as soon as the enclosure E is obtained at step 41, with or without integration of the adapter 5 before such step 42a. In some variants, sterilization is performed just before the bioprinting step 44.

More generally, the method may imply forming/obtaining a flexible part, distributed at least in one amongst the cover part (which may be a sheet part 2) and the side wall SW of the containing part RC and then assembling the flexible part and the other wall members, with a sealing operation at each junction, in order to obtain the sealed enclosure E. The adapter 5 can be already mounted on the cover part, as illustrated in Fig. 3, before the forming 41 of the enclosure E.

The side wall SW may be sized, in order to have an upper annular end 4c thereof larger that an outer diameter of the adapter 5, 105, possibly of at least of same size as an annular lower end joining the base portion 4a. In a variant, the side wall SW may taper upwardly, with provision that the top portion 4b is also adapted to taper in same direction (upwardly) and/or that the side wall SW is directly connected to the adapter 5, 105.

Regarding the base portion 4a, a preliminary step for adapting the inner surface and/or for securing an additional layer or member to the base portion 4a may be involved, in order to improve adhesion of the printed tissue. Indeed, depending on applications (or kind of plastic used for the base portion), adhesion of the printing tissue to the target support may be increased to prevent any part of the printed tissue from moving inside the bioreactor 4. The method may comprise a step of adding or modifying an inner layer at the base portion. A laser treatment or other physico-chemical treatment may be performed, before the sealing to obtain the enclosure E. More generally, a special part at the base portion may be provided to ensure rigidity and/or stability of the target support 20, which may be lying directly on the base portion or be included as an inner layer of the base portion 20.

A recess opening inwardly (inside the enclosure E) may be provided, in order to have the target support 20 delimited and surrounded by projection or an annular wall P20 (see Fig. 3), which extends away from the side wall SW (radially shifted inwardly as compared to the side wall SW). The recess may be obtained by a decrease in thickness of the base portion or of a similar inner layer. Alternatively, the recess may be delimited by an annular projection, possibly formed as a ring, printed by an additive method (3D printing) or suitably/rigidly fastened to the inner layer of the base portion 4a. In other options, the base portion 4a may be thermoformed to have a relief at the target support 20 and/or a similar recess delimitation, with or without an inner projection.

Besides, in some options, the shape of the top portion 4b may be dome-shaped in a by-default configuration, due to thermoforming or similar shaping operation, in order to have the adapter 5 arranged at an apex of such top portion 4b. This may facilitate a sealing step 41 to fasten an annular outer edge E2 of the top portion 4b to other wall members(s) for obtaining the sealed enclosure E, while the depositing end 8 may be already protruding from an inner face of the top portion.

While at least one barbed nozzle or similar connection port, used to form the inlet for nutriments (culture medium) and/or the outlet, is present in at least one of the wall members used to delimit the interior volume V, other connectors may be provided, for instance for circulation of gas such as $CO_2$ and $O_2$ (or filtered air flowing through a HEPA filter). Besides, a temperature sensor and/or a pH sensor may be provided in the interior volume, such sensor(s) being embedded due to a connection (to a wall member of the enclosure E) performed before ending the sealing 41.

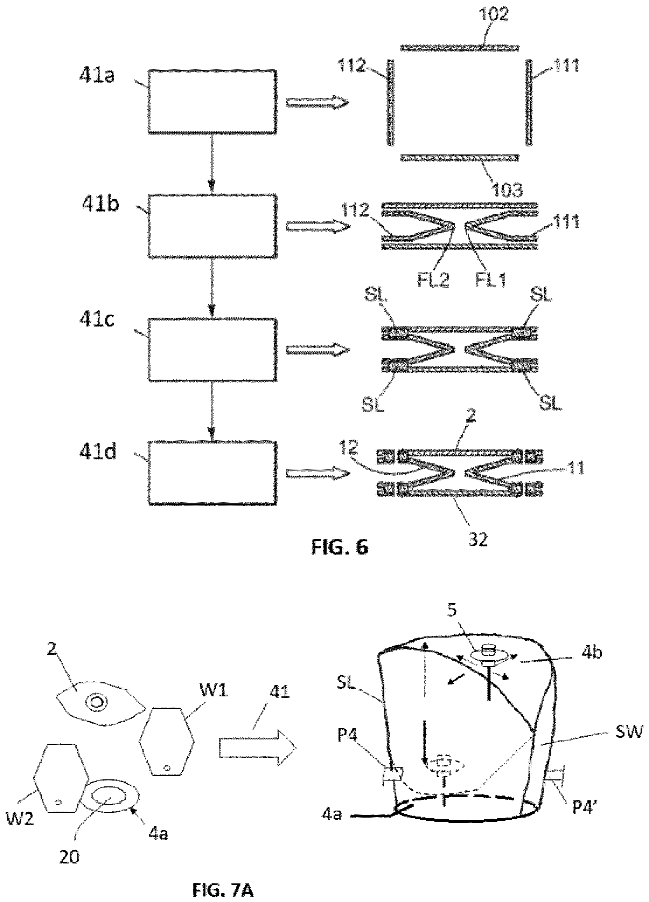

Besides, the connection port(s) P4, P4' can be placed as desired, for instance on a flexible element or on a rigid wall member, possibly at periphery of the base portion 4a as shown in Figs 5A-5B. In Figs 1-2, the connection ports P4, P4' are placed on a rigid side wall but at least one of such connection ports may also be provided in flexible panel-like walls that are flexible, as reflected by Figs 7A-7B.

Possibly, a needle/depositing end 8 for transferring the biological material may be already present before the forming of the enclosure. In order to ensure a sealing without interference with the depositing end 8:

- either such depositing end 8 is prepositioned in a configuration not protruding beyond the lower end of the adapter 5 or protruding less than in an operating configuration,
- or the needle/depositing end 8 is already protruding and the sheet part 2, which is flexible, can be conformed as a dome or similar bulged conformation so that the needle is displaced away from the compartment C1 and does not protrude beyond a fixation region RF.

When providing a rigid base to form the base portion 4a and/or the rigid side wall SW, a silicon seal of annular shape may optionally be sandwiched between two rigid members of an annular shape clipframe. Fig. 5B illustrates such clip frame CF, here when using a deformable sheet 2 to form a top portion 4b, and with one or more connection ports forming the inlet P4 and the outlet P4' secured to a rigid disc D forming all or part of the base portion 4a. Of course, such kind of clip fixture may be used for many other cases, with the bioreactor 4 not necessarily including the ports P4, P4' in such rigid member.

The sealed enclosure E may be delimited by the base portion 4a and a sheet or sheet assembly 2 configured to be dome-shaped, in an expanded state as illustrated in Fig. 5B. The sealing step 41 may involve a welding, a clipping or any suitable way to obtain an annular sealing contact between the peripheral annular region of the assembly 2 and the base portion 4a. A prepositioning step 40a (Fig. 3) may be performed before a welding or similar sealing to obtain the enclosure E. The sheet assembly 2 may be provided with an apex region E14 or similar movable top portion where the adapter 5 is coupled to the sheet or sheet assembly 2. All or part of the steps as illustrated in Fig. 3 may apply to such configuration, especially:

- the coupling step 40 to have the adapter 5 directly coupled to the flexible sheet or sheet assembly 2, ensuring central opening (similar to opening O4) of the sheet assembly 2 is sealed;
- the sealing step 41 to ensure the sheet assembly as a whole, including the apex region E14 or similar top portion provided with the adapter 5, is connected to the base portion 4a;
- the sterilization step 42a to have the sealed enclosure obtained at step 41 sterilized.

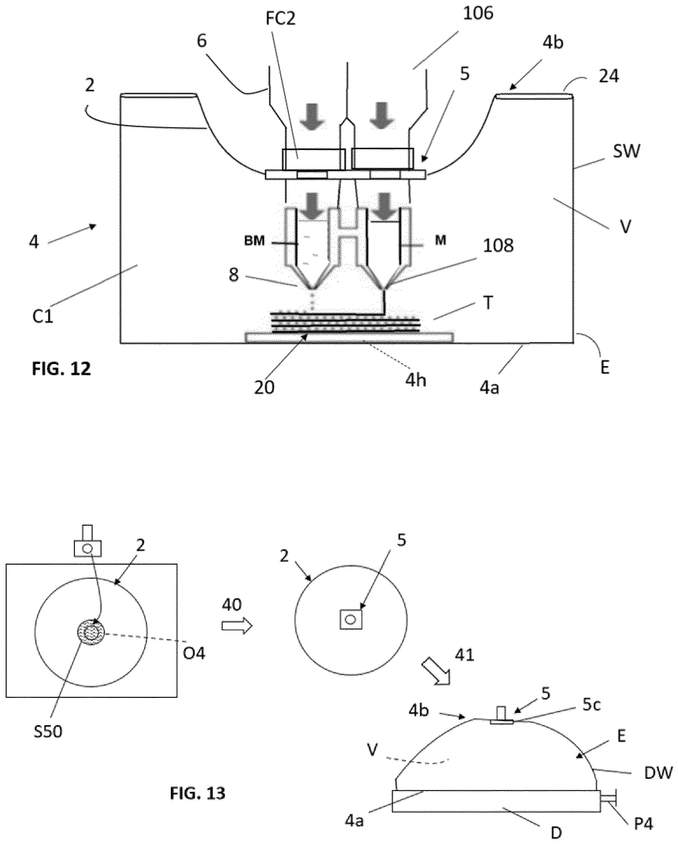

Here, the flexible sheet assembly 2 is provided with an annular border forming an annular end. Referring to Fig. 13, it is shown an embodiment with the sheet 2 made of silicon material or similar flexible plastic, preferably transparent. The raw sheet material may have a rectangular shape, before a cutting operation. An annular cutting can be performed to delimit an outer edge/border of the sheet 2, here with a circular shape, possibly after forming a multilayer region with the sheet 2 coupled to a septum layer, so that the septum layer is already covering the central opening O4. The adapter 5 may include such septum layer to have a septum S50 and a rigid upper guide, provided with a flange 5c, which protrudes upwardly from the multilayer region. The piece including the flange 5c and the tubular guide may be made of silicon, harder (with greater shore hardness) and/or more rigid than silicon material of the sheet 2.

At least one welding operation may be performed at the flange 5c of the adapter 5 to have the adapter 5 (with the septum S50) secured to the region of the sheet 2 surrounding the opening O4. After coupling the adapter 5 at step 40, a sealing may be obtained between the rigid plate or disc D provided with the base portion 4a and the sheet 2, using a clip frame as in Fig. 5B or using any suitable fixation to maintain the annular border in a stationary configuration. The sheet 2 may be inflated due to sterile gas present in the enclosure E after the sealing step 41. All or part of such sterile gas may be introduced in the enclosure E via a gas inlet or port. A dome-shape, a shape close to a sphere or half-sphere may be thus obtained. The enclosure E as shown in Fig. 13 may then be sterilized at step 42a. The disc D or similar base may include/be made of metal or plastic material. The disc or base can be recycled or re-used if needed, provided appropriate cleaning/sterilizing treatments have been provided. In some variants, the sheet 2 may be pre-formed to already have a dome shape, before the sealing step 41 and/or before any prepositioning step 40a.

Such assembly 2 is suitable to taper toward a central portion of the assembly 2, in an expanded state. Such expanded state may be already obtained when performing the connection step 43 to have an outer end of the adapter 5 (extending outside the enclosure E) secured to the movable part (driving system DS) of the external printer unit 3. Of course, the structure of the adapter 5 may greatly vary and is not limited to the example illustrated in Fig. 5B. The adapter 5 just needs to be sealed to the sheet assembly 2 with ability to form a passage for the biological material BM to be deposited via the depositing end 8 that extends inside the interior volume V. The adapter 5 with the flange 5c of annular shape and an upper tube or similar guiding upper end, facilitates guiding of a needle or similar depositing end 8. Such depositing end can easily be inserted in the interior volume V through the opening O4, by piercing the septum S50. After steps 44, 45, the needle may be removed and the septum S50 can, optionally, automatically close the opening O to preserve sealed state.

When the needle or similar depositing end is mounted via the adapter 5 on a flexible sheet assembly that can be dome-shaped, movement along a transverse direction, parallel to the base portion 4a, is facilitated since curvature can vary at different sides of such dome-shaped sheet assembly 2 or 2'. For instance, when moving the adapter 5 toward a given side, possibly without (or without any significant) downward movement, the assembly 2 or 2' can locally increase the curvature at such given side, while at the opposite side a sloppy profile is obtained (for instance with a slope of about 45°). A wall of silicon material that is dome-shaped (wall DW) or having a spheroid shape, i.e. with a curved profile, may easily follow movement of a needle during the bioprinting phase 44, due to flexibility of the material on one hand, and due to absence of any significant/undesirable fold that could impair displacement of the adapter 5. Such sheet assembly 2 is prevented from collapsing due to the coupling of the adapter 5 with the printer unit 3. Also, since gas / air is present in the interior volume V, such lightweight sheet assembly cannot collapse so that no significant fold is formed at or around the top portion 4b. Of course, silicon material is just an exemplary material and any other translucent or transparent resilient plastic having suitable flexibility can be used.

Optionally, one or more cutting steps may be provided, for instance after welding operations to obtain welded seams. Referring to Fig. 6, the sealed enclosure E may be obtained by welding sheets of plastic material and then cutting outer parts. Similarly, a cutting of a peripheral edge of the bottom/base portion may also be performed, possibly after a junction with a side wall SW. While Fig. 3 illustrates a rectangular shape for a sheet 2 coupled to the adapter 5 in a central region, with a cover part 5a of the adapter 5 overlapping the aperture O4, other outer shape may be provided to form outer delimitation of the top portion 4b. A flange may form the cover part 5a, with an axial central passage included in such flange. The respective sides 2a, 2b, 2c, 2d of the sheet 2 may be placed in different faces of the enclosure E when extending in an upstanding/expanded configuration, for instance when flaps are formed. Alternatively, such sides 2a, 2b, 2c, 2d may match with corresponding sides of panels or upper sides of the side wall SW.

In the non-limiting example of Figs 6, 7A and 7B, it can be seen at least the side wall SW is made from several plastic sheets that may be assembled at side edges thereof. Here, in the case of FIG. 6, welded zones are formed for assembling the sheets, with welded zones more rigid than the non-welded parts. Each projecting strip defined by the welded seams may have a maximum thickness which is around two times greater than the thickness in the flexible zones of the flexible side wall SW, making it possible to make the interior volume of the bioreactor 1 vary. In some variants, only a part of the side wall SW has such flexibility, possibly using same kind of assembling as in Fig. 6 or Figs 7A-7B.
The assembly 2' such as shown in Fig. 8A can be associated with the top portion 4b of Figs 7A-7B, in some embodiments.

In some variants, the bioreactor 4 defines a container making it possible a stirring, as described in document EP 2 326 412 (see, in particular, FIGS. 1E to 1H and FIG. 2 in this document).
Besides, when welding flexible sheets or panels, the joins may be produced by a local heating during a sufficiently long period of exposure (which can be of around a few seconds or possibly 10 seconds, for example) to the heat or to a heating by low-voltage electrical impulse (for example, up to 9 impulses), produced by a welding head. The technique of heating by a low-voltage electrical impulse can be used, such that the appearance of the visible face is unchanged, while guaranteeing a good welding quality: indeed, it does not require any high pressure at the time of the welding.

Impulse welding, thermal welding or laser welding techniques can make it possible to obtain resistant welded seams SL. Such welded seams may include longitudinal seams that extend longitudinally at least in the side wall SW. An example using four films for obtaining a 3D-enclosure that can vary in volume due to flexibility of the films is shown in Fig. 4.

After supplying and making available the four films 102, 103, 111 and 112, for instance using rollers (not represented) which unwind these films in one same general direction, called a longitudinal scrolling direction. An assembling may be performed. Of course, this scrolling direction is simply used as a reference point to explain the drawing and it is permitted, of course, to route the films with one or more changes of direction (no need for the transportation direction to correspond to a rectilinear layout).

Referring to Figs 6, 7B and 8B, embodiment examples are given for obtaining side welded seams SL, with or without forming gussets in an initial flat state. Such seams ML may be formed along a longitudinal direction (longitudinal sense or direction which could correspond to the scrolling direction of the film strips) during the production of the enclosure E of the bioreactors 4, here flexible bioreactors.

On the production line, insertion of the gussets 11 and 12 can be done in a manner known per se: see in FIG. 6, the step 41a of supplying and making available the films 102, 103, 111 and 112, and the step 41b of folding the films 111, 112 towards the inside, intended to form the gussets 11, 12. To weld each gusset 11, 12, the corresponding film 111, 112 can be held supported against a guide having been used for folding or against an equivalent abutment element.

Referring to Fig. 8C, it can be seen that gussets G2, for instance a pair of opposite gussets, may also be provided in a sheet assembly 2' that is prepared separate from the receiving part RC of the bioreactor 4, before a final fastening step using the flange 24 of annular shape. Such final fastening step may be performed with a variety of flexible assembly 2, 2' belonging to the covering part of the bioreactor 4. Such sheet part 2 or 2' may form a 3D structure with a wide annular member delimiting a passageway O2 for the depositing end 8, 108 and possibly also for the adapter 5, 105. Such sheet part 2, 2' is adapted to expand with an upwardly tapering geometry (tapering from the annular member/flange 24), and a downwardly tapering geometry when driven downwardly by a driving system DS to have a folding part passing through the passageway O2. The movable flexible part of the top portion 4b may be included in the assembly 2, or comprised (additionally or complementary) in the assembly 2'.

Besides, the adapter 5 may also be secured to the sheet assembly 2' when welding the sheets. As shown in Fig. 8C, the aperture O4 is delimited between two sheets (sheet edges, here). The two sheet edges are distributed in two respective sheets of plastic film material that may sandwich the gussets G2 in a flat state. The two sheet edges are welded at a welding junction SL'. The widening of the sheet assembly 2' is here of interest to improve a degree of freedom when moving the adapter 5. Similarly, the passageway O2 (matching with the access opening O or opened end provided at the top of the side wall SW) may open in a wider space, which is the compartment C1 for the bioprocess (cell maturation or the like).

Film cutting and assembling thus may be involved for at least one amongst the containing part RC and the covering part 2, 2'.

At this stage, some of the connection ports P4, P4' and/or a top aperture O4 or other opening O suitable for accommodating a rigid interface may be already provided, in the main parts of the bioreactor 4: in the containing part RC and in complementary part(s) 2, 2' to cover the containing part RC). Supplying films in an initially planar state may facilitate formation of specific openings and/or mounting of the ports P4, P4'. In variants, the base portion 4a and/or other parts of the containing part RC are made of rigid molded plastic, facilitating integration of nozzles for example for the port(s) P4 and/or P4'.

As illustrated by the non-limiting example of Fig. 6, the four side welded seams SL can simultaneously be made during a welding step 41c, which makes it possible to assemble the four films 102 and 103 (first pair of films opposite one another), 111 and 112 (second pair of films opposite one another and routed transversally with respect to the first pair of films). The folding lines FL1 and FL2 may be kept spread apart during this welding step 41c (defining a minimal transversal space between the first gusset 11 and the second gusset 12 in the flat configuration).

The welded seam length can be adapted. For instance, such length is longer than the final length of the side welded seams SL, in particular in the options which make it possible for oblique cut outs. The pair of films 102 and 103 makes it possible to form, after a cut out step 41d, rectangular sheets from which the respective wall elements 2 and 32 (when the element 32 here forms the base portion 4a) can be obtained. In this non-limiting example, gussets are obtained, which are not also cut out obliquely and which can also be of the same length (along the scrolling direction) as the length of the rectangular sheets. Fig. 8B shows an exemplary expanded configuration of the enclosure E, having here a generally parallelepiped configuration when pressure is not high, due to the gussets 11, 12 being unfolded. The films 102, 103, 111 and 112 may be slightly bent when pressure is increased, for instance if the flexible part 2' to which the adapter 5 is coupled is displaced downwardly with a compressing effect.

The cutting step 41d can be optional. The material of the four films 102, 103, 111, 112 may be here identical. More generally, it is understood that the first wall member 2, the second wall member 32, the first gusset 11 and the second gusset 12 are defined by polygonal sheets, for instance rectangular sheets, optionally having one same multilayer structure, with a layer defining an inner face suitable for the contact with a biopharmaceutical fluid.

The adapter 5 coupled to the flexible film 2' may be welded or sealed, in an annular region surrounding an opening O of the top sheet part 2. With such conception, the side wall SW may possibly include the two opposite flaps 14, 15 of the respective main sheets 2, 4a and the gussets 11, 12. In a variant, the gussets 11, 12 can be involved to form the base portion 4a and the top portion 4b. More generally, orientation of the bioreactor may vary.

Now referring to Figs 7A-7B, the welding may be similar to form different seams, except that no gusset is formed. This reflects another way to perform the step 41 of sealing the wall members. A number of flexible panels W1, W2, 2 are welded to each other such that when the bioreactor 4 is in the upstanding configuration, they form at least the top portion 4b and the side wall SW. For instance, two panels of generally hexagonal shape may be assembled to form seams, possibly similar to the welded seams SL. When all or part of the base portion 4a is flexible, radially protruding external extensions or similar fixing extensions may already be provided around/along an outer circumference of the base portion, before the sealing. In a variant, the base portion is rigid at an outer peripheral part thereof, is stiffened, o is stabilized externally, by a pulling at opposite ends thereof.

Such kind of assembling is suitable to combine several flexible parts without integration of rigid members in the enclosure E, except possibly the base portion 4a. While Fig. 7A-7B shows an adapter 5 designed as a needle holder, plugging the aperture O4 of the top sheet 2 used to form the top portion 4b, another way of forming a flexible part moving with the adapter 5 may be provided.

For instance, a relatively wide opening O as in Fig. 8 B may be provided in the sheet part 2, allowing mounting a rigid coupler 56 of tubular shape and then, a movable depositing end assembly 2', including a flexible film having the aperture O4 plugged by the adapter 5 and an outer edge sealed and rigidly fastened to the coupler 56, can be mounted to be movable through the access of the opening O. Here, the assembly 2' is constructed as an additional wall member of the covering part 2 (extending the covering part 2 to plug/seal the opening O delimited by the coupler 56).

Referring to Fig. 8A or 8C, the assembly 2' is a needle single use system, which may be sterile and closed. Material (stretchable material or non-stretchable) of the sheet(s) involved in this assembly 2' may be transparent, forming a film for instance. In the example of Fig. 8A, such sheet may be attached to the adapter 5 at an annular inner edge delimiting the aperture O4, and optionally attached annularly to an annular flange 24 secured to a sheet outer edge (also of annular shape). The periphery of the assembly 2' may have a shape that remains unchanged, for instance due to rigidity of the flange 24 that delimits an outer perimeter of the assembly 2' (at least in a substantially flat configuration of the assembly 2').

Referring to Figs. 8A to 8C, the coupler 56 may be coupled to the flange 24 or directly to the sheet, at an annual outer edge. Before such coupling, the passageway O2 may be hermetically closed, for instance by a suitable membrane 26 in annular sealing contact with a face of the annular flange 24. Accordingly, it is provided a needle single-use system (here assembly 2') that is sterile and closed. Of course, any of the other sheet assemblies, including the side wall SW or not, may have such membrane 26 if needed (see for instance Fig 5B and Figs 9-12) to protect a corresponding compartment C1 and/or C2.

The flange 24 may be involved in any one of the following exemplary connections: luer lock fitting from the needle or assembled on the needle; triclamp port, screw port or aseptic connection at an annular end of the side wall SW.

When having a wide opening O giving access to the lower/main compartment C1 of the sealed enclosure E, any suitable depositing end assembly 2' may be preassembled with a flexibility of the sheet material thereof that surrounds the adapter 5 that bears the needle/nozzle forming the depositing end 8. Such preassembled assembly 2', here forming an upper compartment C2 when extending/protruding upwardly, can be hermetically fastened to the frame or similar coupler of annular shape provided at the top of a 3D-bag including the base portion 4a and obtained by welding one or of sheets of film material forming at least the side wall SW. A sealed enclosure E is obtained after sealing the assembly 2, 2' onto the opening O and/or above the side wall SW, with the assembly 2, 2' having suitable mobility to be inserted in part in the lower compartment C1, as illustrated in Fig. 8B (same logic applies for Fig. 10 or 12, without limiting the possibilities).

Since the target support 20 is already present at the base portion 4a, while suitable connection ports P4 and/or P4' are present, the sealed enclosure E obtained at step 41 is directly a bioreactor 4, provided with a movable assembly 2' to allow the bioprinting at step 44.

**EXAMPLARY OUTER CONNECTION PROVIDED BY THE ADAPTER**

So far, the adapter 5, 105 has been described as a coupler (bearing the needle or similar depositing end 8) arranged at the opposite from the base portion, plugging an aperture O4 that is centered in a top portion 4b of the bioreactor 4, possibly forming a septum S50. More generally, the adapter 5 may be provided at any suitable position on the enclosure E, away from the base portion 4a and compatible with movement of the driving system DS above the target support 20 that is provided on or above the base portion 4a.

The adapter 5, 105 may be provided with an outer connecting part FC1 that is preferably rigid. Referring to Fig. 2, 4A or 12, the sealed enclosure E may be obtained before fixing a cartridge 6, 106 to the adapter 5, 105, preferably using the outer connecting part FC1. The cartridge 6, 106 is designed to contain all or part of the material to be printed and, optionally, serve as a mechanical interface with the driving system DS, using fastening means 6f of any suitable form. Such means may be mechanically and/or magnetically fastening means, for instance.

Such cartridge 6, 106 may be kept substantially vertical when connected to the adapter 5, 105 and being in fluid communication with the needle or similar depositing end 8. In some variants, such cartridge 6 may already be connected to the adapter 5, possibly being a part of the top portion 4b when fastening the top portion to the wall member(s) of the containing part RC. When the adapter 5 is already present in the top portion 4b without the cartridge 6, such cartridge 6, 106 may be supplied and sterilized separately and connected to the printing needle/depositing end 8, 108 via the adapter 5, 105. Here, the adapter can be arranged at an apex of the dome-shaped top portion, which may be a storage position during a bioprocess with culture media entering the compartment C1 of the interior volume V, to interact with cells of printed tissue T.

The adapter 5, 105 may be preassembled with a needle or similar depositing end 8, 108 protruding with respect to the lower face of the adapter, before being coupled to the sheet part 2, 2' and/or before being included in a similar upper part covering the compartment C1 from above. In some options, a molding step or overmolding is performed to have the needle included in a piece forming an outer body of the adapter. A central/longitudinal channel may thus be provided in such piece, at least at the depositing end 8 for flowing of biological material BM, M. At the opposite from the depositing end 8, the adapter 5, 105 may be provided with an inlet part, forming a first longitudinal end of the adapter 5. In some options, an anti-return valve or similar automatic closure member may be provided at the first longitudinal end of the adapter. The second end may be nozzle-shaped to reduce the diameter, allowing delivery of a flow of reduced section. The closure member may be actuated when pressed (for instance pressed downwardly) by a pushing member belonging to the cartridge unit provided with at least one cartridge 6, 106. The cartridge may be a syringe with a piston. A return member (elastic return means such as a helical spring or the like) may be provided, allowing automatic closure when the cartridge is disconnected. In some options, two cartridges or more may converge toward a same feeding channel, cooperating with same inlet part of the adapter outer connecting part FC1.

Referring to Fig. 9, a needle or similar biological material depositing end 8 may be secured to an in inserted part of the adapter 5, here configured as a connector allowing fluid communication with a cartridge 6 when two connecting parts FC1, F2C are assembled and locked together. The cartridge 6 is provided with a connecting part FC2 that extends around a fluid passage opening at an axial aperture O6. In the adapter 5, the outer connecting part FC1 may have a single piece made of molded plastic material, provided with a tubular part delimiting the fluid passage. For instance, material of the connecting parts FC1, FC2 is not compressible and thus cannot deform.

A connecting device is here obtained when coupling the connecting parts FC1, FC2 provided in the attachment components 25, 25', using a mechanical fastening. In embodiment of Fig. 9, the connecting parts FC1, FC2 are provided entirely outside the interior volume V and include each a respective annular part, optionally with a complementarity in shape. The annular part of the first connecting part FC1 provided in the adapter 5 may be radially fastened, fixedly, to the central tube section of the facing annular part provided in the second connecting part FC2. The free end of this central tube section defines the aperture O6 (second opening facing the access opening of the adapter 5). An end flange or an annular rim is provided around this aperture O6, to form a receiving surface to which a membrane SM' covering the aperture O6 is detachably adhered.

Similarly, the membrane SM covers the access opening of the adapter 5. Each membrane SM, SM' may be (optionally folded and unfolding may be performed when attaching the two connecting parts FC1, FC2 via their attachment component 25, 25'. The connecting parts FC1, FC2 may be of the kind having a female component (attachment component 25 as illustrated in Fig. 9) cooperating with the male component (attachment component 25' shown in Fig. 9), using a locking slider and clips or similar locking members.

Exemplary comparable sliding connections are of the range Opta® SFT sterile connectors from the Applicant. The connecting parts FC1, FC2 allow fast and reliable sterile connection and sterile fluid transfer between two separate, pre-sterilized process components in biopharmaceutical manufacturing operations. Each membrane SM, SM' may be a sterilizing grade Polyethersulfone (PES) membrane or similar. The couplings or attachment components 25, 25' involved in locking the connection may be provided with foolproofing means, for instance requiring a sliding or similar guiding along a direction perpendicular to the longitudinal direction of the adapter 5 (formed at the coupling region or requiring a rotation along a longitudinal axis of the adapter). Here, the depositing end 8 is illustrated as an elongated needle or the like, preferably rectilinear and extending along the longitudinal axis A1 of the adapter 5. But other configurations for such depositing end 8 may be adapted, for instance with a deviation angle and/or a bending.

Regarding the membranes SM, SM', they may be configured to be coupled, for instance at a pulling end (becoming a common pulling part in some options, combining the pull members 5b), thus allowing simultaneous removal of the two membranes SM, SM'. Such coupling step for associating the membranes may be performed before locking the connection between the components 25, 25'. Of course, the structure of the outer connecting part FC1 may vary (with or without use of such membranes SM, SM'), and a locking may be performed, possibly using a separate piece. Typically, the cartridge connection allowed by the adapter 5, 105 may be suitable for a single-use situation, the depositing end 8, 108 not being intended to be used with other cartridge(s) after the bioprinting.

A printing unit 3 is obtained as the cartridge 6, 106 may be driven by a driving system DS, according to a program, with movement of a cartesian 3D printer. The adapter 5 is arranged as an end of the cartridge part, which protrudes in the interior volume V. The adapter 5 is a printhead for biological deposition, deprived of any pumping part and, optionally deprived of any swivel or joint J3, which are included in the external driving system DS (entirely extending outside the enclosure E).

## APPLICATION OF THE BIORECTOR AS A SINGLE-USE VESSEL

As above disclosed, the top portion 4b may be arranged as a flexible (typically transparent) plastic part, formed as at least one funnel, directly on the side wall SW or mounted on an annular frame/interface arranged on a sheet part covering the side wall SW. The bioreactor 4 may be produced with one or more functional components 30 already extending inside the interior volume V. A connector 30c associated to such component/sensor is arranged at an outer interface and may be connected to a control unit 35 of the printer unit 3. Whatever the flexibility is obtained in the enclosure E, it is especially adapted to be sterilized.

The sterilizable sealed enclosure E is provided with a first port P4 and/or a second port P4' distinct from the aperture O4 plugged by the fitting/adapter 5, 105. Such ports P4, P4' allow forming a fluid inlet and a fluid outlet. The sterilizable sealed enclosure E has an interior face compatible for containing a biopharmaceutical product and is adapted for carrying on the two following steps:

-    automated, preferably layer-by-layer, printing of bio-ink materials (step 44 - Fig. 3);
-    carrying out maturation or cell culture of the printed tissue/part T (step 45).

A sterilizing step may be performed, for instance before the step 42, ensuring the bioreactor 4 is usable for the bioprinting step 44. More generally, such sterilization step 42a may be performed before the bioprinting phase 44 and possibly before some preparation steps 42, 43 related to the bioreactor 4. In some options, the sterilization step 42a is performed in a collapsed state of the bioreactor 4. Alternatively, a sterilizing operation is performed in an expanded state of the bioreactor 4. Of course, the step 43 of coupling the adapter may be performed before the step 42.

Referring to Figs 1-3, the enclosure E may be firstly mounted in an operating configuration, in which the base portion 4a is stabilized to have a stationary target support 20 and/or connected to a holding and retaining structure HR to limit/prevent any collapsing effect at the side wall SW of the bioreactor 4. This stage is suitable when using a side wall SW that can deform. At this stage, a spacing step 42 is performed for spacing two opposite portions of the flexible side wall SW, possibly using several fixation members MF'. In any case, the side wall SW is constructed or assembled with such structure HR, in order to be kept with a substantially annular shape, in upstanding configuration of the bioreactor 4.

Fig. 8D illustrates an option, in which step 41 provides a welding junction/seam SL that improves stability at an upper annular end 4c of the side wall SW, possibly without any holding contact except at the base portion 4a (via the fixation means MF). The sealed enclosure E may be formed as a D2 bag (here with only two sheets of flexible plastic material, without gussets), with a soft or rigid support on/at the bottom surface. A preforming treatment may be selectively performed to stiffen the bottom forming the base portion 4a. Of course, the outer perimeter may be of any shape at the bottom and at the top of the annual side wall SW, in upwardly standing configuration of the bioreactor 4 (circular, square other polygonal shape). The port(s) P4 may be welded on any suitable enclosure surface, for instance on the bottom or in the seams SL. The plastic film may be chosen so that the seam SL prevents the sheet part 2 to collapse in a by-default configuration, should the adapter 5 be disconnected from the driving system DS. After the bioprinting, the adapter 5 and the depositing end 8 may be raised as illustrated, so that they do not interfere with a bioprocess performed in the compartment C1 of the enclosure E.

While Fig. 8D, shows a particular embodiment compatible with a reduction in the number of flexible parts to be assembled, another kind of enclosure may be obtained, using a side wall SW and a top portion 4b that are tapering in opposite directions (toward the base portion 4a and the adapter 5, respectively), at least in an upstanding configuration of the bioreactor with the upper sheet assembly (delimiting the compartment C2) upwardly expanded. Besides, a groovy profile may be applied for the top portion 4b, with grooves (not shown), possibly with concentrical disposition of the grooves. Grooves may optionally be obtained by thermoforming at least one sheet of the top portion 4b, possibly with a by-default geometry which is substantially flat or dome-shaped. Unfolding at the grooves may help in displacement of the adapter 5, 105 inside the interior volume V.

The choice of spacing means may vary, for instance to provide a stretching effect. In some options, the spacing means are under the form of stiffening parts or retaining members of the structure HR. Such spacing means prevent the two opposite portions of the side wall SW from moving inwardly. Accordingly, it prevents contact between the target support or the adapter 5 and the side wall SW, while the adapter 5 is moved in an interior volume region, above the target support 20. When the spacing is performed, a periphery of the top portion 4b may be prevented from moving, or only with a small extent, inferior to 5 mm for instance.

When starting step 44 or before such step, the cartridge 6, 106 is connected to the bioreactor 4, selectively using the adapter 5 that may be needle-free or provided with a needle. This is a cartridge - adapter connection step 43. At such step 43, each depositing end 8, 108 is connected to a corresponding microfluidic pump and/or a pump allows the biomaterial BM, M to be pushed downwardly to reach the depositing end 8, 108.

A robotic arm can be equipped with one or more cartridges 6, 106. In some embodiment two/several robotic arms are provided when more than one cartridge is involved in the bioprinting step 44. In some options the bioprinting step may involve printing multiple cell types. The robotic arm such as illustrated in Fig. 2 or 10 is here given as a non-limiting example for the driving system DS. Any mechanical with movable parts assembled for relative displacement of the printhead/depositing end 8, 108 rigidly secured to the adapter 5, 105 with respect to the target support 20 may be provided.

The robotic arm or similar system DS can move according to the 3D-design in X, Y and Z direction to print the tissue, according to instructions of the control unit 35. Depending on the programming of the printing parameters of the bio-inks (containing cells and/or biomaterials), it is permitted defining certain physical parameters of the printer unit 3 and the trajectory of the depositing end(s) 89, 108 forming the embedded printhead(s).

In embodiments using a flexibility of the material at the top of the enclosure E, the portion 4b can surround an arm or similar part of the driving system DS, at least during bioprinting. In other embodiments compatible with a rigid plate or similar rigid top portion 4b covering the containing part RC, flexibility of the side wall SW is such that it compensates lateral movement of such top portion 4b, as in Fig. 5A. Dotted lines in Fig. 5A reflect change of conformation and displacement of the external holders 51, thus allowing a lowering of the position of the depositing end 8. The top portion 4b may be significantly sloped/tilted, without varying position of the base portion 4a or position of the region thereof, where the target support 20 is provided.

In embodiment of Fig. 5B, a pressure management in the enclosure E may be performed, optionally using at least one port 55a, 55b separate from the port(s) P4, P4'. The flexible wall of the enclosure E may be a dome-shaped wall DW, at least in an expanded state, adapted to taper from a lower periphery of the base portion 4a or from a side wall part. Such wall DW may be resilient, either deformable at constant surface or able to increase its surface. Such wall may be controlled to collapse sufficiently to allow displacement of the adapter 5, which includes a septum S50 or (alternatively) a channel for accommodation or communication with a needle/depositing end 8.

In these embodiments of Figs 5A and 5B, while only one depositing end 8 is shown, it is understood that at least two depositing ends may extend in same interior volume. Connection step 43 may be performed to have a respective cartridge or cartridge reservoir part associated to the corresponding depositing end, using same adapter 5.

Referring to Fig. 3, the connection step 43 may occur, either before the optional spacing step 42 or after the spacing step 42. The cartridge 6, 106 may be filled at a later stage in some options, i.e. after the connection step 43 and before the bioprinting step 44. Before or at the connection step 43, a depositing end 8 is provided, possibly inserted through the aperture (O4) when secured to the adapter 5. As a depositing end 8 is extending inside the enclosure E from the adapter 5, the biological material BM or M contained in the reservoir of the cartridge 6, 106 may be deposited on the target support 20, once the adapter 5 is displaced in the accurate position with the depositing end 8, 108 facing/reaching the target support 20.

During the bioprinting at step 44 to produce a tissue T, the adapter 5, 105 and the depositing end 8, 108 are moved together inside the interior volume V relative to the target support 20, along at least two transverse directions. Such transverse directions may be perpendicular to Z direction and perpendicular to direction of longitudinal axis A of the adapter 5, 105. Regarding movement along Z direction, flexibility of the enclosure upper part and/or flexibility provided at the side wall SW may be sufficient to move and lower the depositing end 8, 108 at a different height level, and also raising the depositing end 8, 108. A storage step for storing the depositing end may be involved after the step 44 and before the step 45. Alternatively, a welding and possibly cutting may be performed to isolate and extract the depositing end 8, 108 away from the receiving part RC. In such case, the welding allows hermetically sealing at least the lower compartment C1. A pinching may also be used instead of a welding.

Since the interior volume V is already closed, the tissue T may be submitted to a bioprocess, for example a maturation step 45 or similar phase with cell culture. The port P4 and/or P4' provide suitable introduction and circulation of a culture media, flowing in the containing part RC. Functional components 30 already present in the interior volume V, such as sensors, are available to deliver suitable signal/information reflecting key parameters of the bioprocess, while the tissue remains in a position on the support 20. Also, a stirring member 29 may be activated as the base portion 4a may have a suitable interface, possibly using a magnetic interaction. The stirring member 29 may be associated to an interface provided in the base portion 4a or below the base portion, allowing introduction of a magnetic source adapter for a magnetic coupling with the stirring member.

Regarding the printer unit 3, which is an external assembly, arranged outside the bioreactor 4, any suitable feed source may be included for feeding the nozzle/needle or similar depositing end 8, 108 with the biological material BM, M. The feed source may consist of or comprises a reservoir and a pump (feed pump, possibly with a pressurizing effect). Preferably, activation/actuating means for activating the transfer of the biologicals objects to the target support are entirely outside the bioreactor 4. Such activation/actuating means may be a control part for feeding the depositing end 8, 108 for example, a mechanical control part by a worm drive, or a piston, or a direct pneumatic control part.

It is understood the different steps 44, 45 (possibility with several bioprinting cycles) take place in same bioreactor 4, for instance to allow the cells to self-organize, possibly until the specific biological functions (which are sought) are obtained/emerge. After the last cell culture (end of maturation step 44), the printed matter/tissue T may be retrieved. After such step 44, the bioreactor 4 including the adapter 5, 105 is optionally removed from a support and/or disconnected from the holding and retaining structure HR and then, the bioreactor 4 is discarded. A disconnection to remove the cartridge 6, 106 may also be performed, for instance before discarding the single-use bioreactor 4.

Of course, the interior volume V may vary depending on the applications and the enclosure shape may be suitably adapted. For instance, height may be increased and or a width to have a better degree of freedom. It will be understood by those skilled in the art that other variations and modifications can easily be made within the scope of the invention as defined by the appended claims, thus it is only intended that the present invention be limited by the following claims.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

**Claims**

1. A method of assembling a bioreactor (4) allowing bioprinting inside an interior volume (V) of the bioreactor using a biological material depositing end (8, 108) for delivering a biological material (BM, M) on a target support (20), the method comprising:

- providing the target support (20) for the bioprinting, on or above a base portion (4a) of the bioreactor (4);
- coupling (40) an adapter (5, 105) to a bioreactor top portion (4b; 2') having an aperture (O4), the adapter (5, 105) hermetically plugging the aperture (O4);
- forming a side wall (SW) made integral with the base portion (4a) to delimit the interior volume of the bioreactor (4), the side wall (SW) being adapted to taper and/or extend longitudinally, upwardly away from the base portion (4a) in an upstanding configuration of the bioreactor;
- providing at least one connection port (P4, P4') for direct fluidic communication with the interior volume (V), in order to form a fluid inlet and a fluid outlet, so that the bioreactor (4) is usable for a culture and/or maturation phase of the printed material inside the interior volume (V), after the bioprinting (44); and
- forming (41) a sealed enclosure (E) including the side wall (SW), by at least one sealing operation to interconnect the base portion (4a) and the top portion (4b; 2'), with the sealed enclosure (E) being at least partly flexible and movable, in order to allow a relative movement between the adapter (5, 105) and the target support (20), so that the biological material depositing end (8, 108), inserted through and/or adapted to be fed via the aperture (O4) when secured to the adapter (5, 105), is movable inside the interior volume (V) relative to the target support (20) along at least two transverse directions.

2. The method according to claim 1, wherein the at least one sealing operation is performed before any connection of the adapter with a driving system (DS), preferably before any connection between the adapter (5, 105) and a cartridge (6, 106) or reservoir containing the biological material (BM, M).

3. The method according to claim 1 or 2, wherein the adapter (5, 105) is positioned distally from the side wall (SW), the method comprising:

- forming the top portion (4b; 2') using at least one flexible sheet of film, so that the interior volume (V) is variable in the upstanding configuration, at least as a function of a conformation of the top portion (4b; 2').

4. The method according to claim 3, wherein the top portion (4b; 2') is provided with a deformable surface that is sized, so that in the upstanding configuration the top portion (4b) is movable from an intermediary folded state, which is preferably a flat state, to:

- an outwardly orientated expanded state that increases the interior volume (V), with the top portion (4b; 2') being dome-shaped so that the adapter (5, 105) extends above the side wall (SW) in said upstanding configuration, and
- an inwardly bent state that reduces the interior volume (V), with the top portion (4b; 2') forming a bioreactor recess for passage of a driving system intended to be connected to the adapter (5, 105), while the adapter is surrounded by the side wall (SW).

5. The method according to any one of the preceding claims, comprising:

- maintaining an upper end (4c) of the side wall (SW) in an annular conformation by spacing means directly coupled to a flexible portion of the sealed enclosure (E) which surrounds the adapter (5, 105), the flexible portion forming all or part of the top portion (4b; 2');
- configuring the adapter (5, 105) as a rigid connectable end part or extension of a driving system (DS) arranged outside the bioreactor (4), whereby the flexible portion can be displaced and follow movement of the adapter (5, 105) when the bioprinting is performed with delivery of the biological material (BM, M) on the target support (20).

6. The method according to any one of the preceding claims, comprising:

- sealing a number of flexible panels (W1, W2; 7, G2; 11, 12, 2, 32) to each other such that when the bioreactor (4) is in the upstanding configuration, they form at least the top portion (4b) and the side wall (SW); and
- providing, in the base portion (4a) that is at least partly flexible, radially protruding external extensions (MF) adapted for holding the base portion (4a) substantially planar or flat, along a base plane (P).

7. The method according to any one of the preceding claims, comprising:

- before the sealing operation to interconnect the base portion (4a) and the top portion (4b; 2'), fabricating and/or mounting a receiving surface on an inner face of the base portion (4a), which delimits the interior volume (V), in order to define all or part of the target support (20);
- and then selectively use a peripheral margin of the base portion (4a), around the receiving surface, for fastening with a complementary part, in order to obtain the sealed enclosure (E), the complementary part including the top portion (4b) and having flexible material to delimit the interior volume (V) from above.

8. The method according to any one of the preceding claims, comprising varying capacity of the interior volume (V) due to movement of the top portion (4b; 2') toward a position, in which the top portion (4b; 2') is collapsed and adapted to be downwardly pressed by the adapter (5, 105) when the adapter is driven from outside by a driving system (DS).

9. The method according to claim 1 or 2, wherein the adapter (5, 105), which is preferably a needle holder, is permanently connected with or to a base material of a flexible plastic sheet forming all or part of the bioreactor top portion (4b; 2').

10. The method according to any one of the preceding claims, wherein the side wall (SW) is obtained by welding at least two pieces, at least one of which is:

- made of a plastic film (7, G; 102, 103, 111, 112; W1, W2), preferably a multilayer film, and
- provided with the fluid inlet port (P4) and/or the fluid outlet port (P4').

11. The method according to any one of the preceding claims, comprising a spacing step (42) for spacing two opposite portions of the side wall (SW), which is of annular shape in the upstanding configuration of the bioreactor, by spacing means, preferably under the form of stiffening means or retaining means, whereby the spacing means prevent said two opposite portions of the side wall (SW) from moving inwardly, toward one another.

12. The method according to claim 1 or 2, wherein all or part of the top portion (4b) is made of rigid material and is configured to be movable due to flexibility of the side wall (SW), the method further comprising spacing the top portion (4b) from the base portion (4a) using return members, made separate from the side wall (SW), the return members being coupled to the top portion and exerting an upwardly biasing force, whereby the top portion is by default spaced from a predetermined distance from the base portion (4a).

13. The method according to any one of the claims 1-3, wherein the top portion (4b; 2') is made flexible, preferably of a flexible film, rendering the adapter (5) displaceable with respect to the base portion between:

- a first position, distal from the base portion (4a), in which the adapter (5) is surrounded by a flexible part of the top portion (4b; 2') that tapers upwardly or is dome-shaped and protrudes upwardly,
- and a plurality of second positions, in which the adapter (5) extends proximal relative to the base portion (4a) with the flexible part folded downwardly.

14. The method according to claim 13 comprising varying a pressurizing level in the interior volume (V), in order to move the top portion (4b) between a collapsed state and an expanded state, the expanded state allowing the adapter (5, 105) to be moved above the target support (20), preferably by driving a robotized arm arranged outside the bioreactor (4), due to flexibility of a plastic material forming the top portion (4b; 2').

15. The method according to any one of the preceding claims 1-4, comprising:

   - a first connection step for connecting the side wall (SW), which includes or is a rigid member of generally annular shape, to the base portion (4a); and
   - a second connection step for connecting the side wall (SW) to the top portion (4b) that is made of at least one flexible plastic sheet (2).

16. The method according to any one of the preceding claims, comprising severing an uppermost part of the top portion (4b; 2'), after the bioprinting and after welding two opposite portions of the top portion (4b; 2') at a welding area, in order to reduce the interior volume (V), the severed uppermost part entirely extending above the welding area.

17. The method according to claim 1, comprising:

   - at the opposite from the base portion (4a), hermetically fastening a rigid coupler (56) of annular shape to a flexible sheet (2) of material adapted to delimit the interior volume (V) from above and adapted to form one or more flaps (14, 15) belonging to the side wall (SW), the side wall (SW) being made of a plurality of flexible sheets;
   - sealing a sheet assembly (2') to the rigid coupler to cover an opening (O) of the rigid coupler, the sheet assembly (2') having an outer edge or flange (24) directly fastened to the rigid coupler (56);
   - sizing a top covering surface of the sheet assembly which extends above the rigid coupler (56), so that said top covering surface is adapted to be folded inwardly and outwardly, respectively, in relation to the rigid coupler (56), whereby the adapter (5, 150) can move along the at least two transverse directions below the rigid coupler (56) without any driving part contained in the interior volume (V).

18. The method according to any one of the preceding claims, comprising connecting a mobile member (29) of a stirring apparatus to the containing part (RC), so that the mobile member (29) extends inside the interior volume (V) after forming (41) the sealed enclosure (E).

**FIG. 1**

**FIG. 2**

**FIG. 3**

**FIG. 4A**

FIG. 4B

FIG. 4C

FIG. 5A

FIG. 5B

**FIG. 6**

**FIG. 7A**

FIG. 7B

FIG. 8A

EP 4 316 843 A1

FIG. 8B

FIG. 8C

34

**FIG. 8D**

**FIG. 9**

**FIG. 10**

**FIG. 11A**

**FIG. 11B**

FIG. 12

FIG. 13

Europäisches
Patentamt
European
Patent Office
Office européen
des brevets

# EUROPEAN SEARCH REPORT

Application Number

EP 22 30 6181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/269503 A1 (BORN JOHANNES [DE] ET AL) 27 August 2020 (2020-08-27)<br>* paragraph [0035] – paragraph [0047] *<br>* paragraphs [0049] – [0053] *<br>* figures 1-4,6-9 * | 1-18 | INV.<br>B33Y70/00<br>B29C64/106<br>C12M1/26<br>C12M1/00<br>C12M3/00 |
| A | US 2019/184639 A1 (LOPEZ ROMANO BERNARDO [ES] ET AL) 20 June 2019 (2019-06-20)<br>* paragraph [0028] – paragraph [0029]; figures 6,7 * | 1-18 | |
| A | US 2016/361873 A1 (MAIER NATHAN CHRISTOPHER [US])<br>15 December 2016 (2016-12-15)<br>* paragraph [0009] *<br>* paragraph [0031] – paragraph [0047] *<br>* figures 2,4-6 * | 1-18 | |
| A | US 2019/256270 A1 (BAZIN FRÉDÉRIC [FR])<br>22 August 2019 (2019-08-22)<br>* paragraph [0002] *<br>* paragraph [0141] – paragraph [0150] *<br>* figure 3 * | 6,10,16 | |
| A,D | EP 2 326 412 A1 (SARTORIUS STEDIM BIOTECH SA [FR]) 1 June 2011 (2011-06-01)<br>* claim 1; figures 1A-1H,2 * | 18 | TECHNICAL FIELDS SEARCHED (IPC)<br><br>B33Y<br>B29C<br>C12M |

The present search report has been drawn up for all claims

| Place of search | Date of completion of the search | Examiner |
|---|---|---|
| Berlin | 12 January 2023 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 30 6181

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2023

| Patent document cited in search report | | | Publication date | Patent family member(s) | | | Publication date |
|---|---|---|---|---|---|---|---|
| US 2020269503 | A1 | | 27-08-2020 | CN | 111619104 | A | 04-09-2020 |
| | | | | DE | 102019202660 | A1 | 27-08-2020 |
| | | | | EP | 3702129 | A1 | 02-09-2020 |
| | | | | US | 2020269503 | A1 | 27-08-2020 |
| US 2019184639 | A1 | | 20-06-2019 | CN | 109940885 | A | 28-06-2019 |
| | | | | EP | 3501799 | A1 | 26-06-2019 |
| | | | | US | 2019184639 | A1 | 20-06-2019 |
| US 2016361873 | A1 | | 15-12-2016 | US | 2016361873 | A1 | 15-12-2016 |
| | | | | US | 2019381723 | A1 | 19-12-2019 |
| US 2019256270 | A1 | | 22-08-2019 | CN | 109476404 | A | 15-03-2019 |
| | | | | EP | 3481737 | A1 | 15-05-2019 |
| | | | | FR | 3053586 | A1 | 12-01-2018 |
| | | | | US | 2019256270 | A1 | 22-08-2019 |
| | | | | US | 2021122553 | A1 | 29-04-2021 |
| | | | | WO | 2018007691 | A1 | 11-01-2018 |
| EP 2326412 | A1 | | 01-06-2011 | AT | 556764 | T | 15-05-2012 |
| | | | | EP | 2326412 | A1 | 01-06-2011 |
| | | | | FR | 2933881 | A1 | 22-01-2010 |
| | | | | US | 2011158037 | A1 | 30-06-2011 |
| | | | | WO | 2010007273 | A1 | 21-01-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220105686 A1
- WO 2022064125 A1
- EP 3194150 B1
- US 20200406542 A
- EP 2326412 A